# EUROPEAN PATENT SPECIFICATION

(11) **EP 3 383 405 B1**
(45) Date of publication and mention of the grant of the patent: **12.06.2024**
(21) Application number: 16805745.3
(22) Date of filing: 24.11.2016
(51) Int. Cl.: A61K 31/704, A61P 31/04, C12N 1/20, C12P 29/00

(54) **ANTIBIOTIC FIIRV 104/18 COMPLEX AND THE ISOLATED INDIVIDUAL FACTORS THEREOF**
ANTIBIOTISCHER FIIRV 104/18-KOMPLEX UND ISOLIERTE INDIVIDUELLE FAKTOREN DAVON
COMPLEXE ANTIBIOTIQUE FIIRV104/18 ET FACTEURS INDIVIDUELS ISOLÉS DE CELUI-CI

(30) Priority: 02.12.2015 EP 15197443
(43) Date of publication of application: 10.10.2018
(73) Proprietor: Fondazione Istituto Insubrico di Ricerca per la Vita, 21040 Gerenzano (VA) (IT)
(72) Inventor: CARRANO, Lucia, 20025 Legnano (MI) (IT); ABBONDI, Monica, 22060 Cabiate (CO) (IT); CARENZI, Giacomo, 21052 Busto Arsizio (VA) (IT); BRUNATI, Mara, 22077 Olgiate Comasco (CO) (IT); SIGURTA', Alessandro, 21047 Saronno (VA) (IT); TARAVELLA, Anna, 21047 Saronno (VA) (IT); TURCONI, Paola, 21053 Castellanza (VA) (IT); CANDIANI, Gianpaolo, 20064 Gorgonzola (MI) (IT)
(74) Representative: Manfredi, Irene
(86) International application number: PCT/EP2016/078678
(87) International publication number: WO 2017/093114

(56) References cited:
- JP-A- H0 196 189
- US-A1- 2012 010 163
- RATNAKAR N ASOLKAR ET AL: "Arenimycin, an antibiotic effective against rifampin- and methicillin-resistant Staphylococcus aureus from the marine actinomycete Salinispora arenicola", THE JOURNAL OF ANTIBIOTICS, vol. 63, no. 1, 20 November 2009 (2009-11-20), pages 1-10, XP055082155, ISSN: 0021-8820, DOI: 10.1038/ja.2009.114 cited in the application
- ANASTASIJA REIMER ET AL: "Inhibitory activities of the marine streptomycete-derived compound SF2446A2 against Chlamydia trachomatis and Schistosoma mansoni", THE JOURNAL OF ANTIBIOTICS, vol. 68, no. 11, 20 May 2015 (2015-05-20), pages 674-679, XP055270718, GB ISSN: 0021-8820, DOI: 10.1038/ja.2015.54
- R. D. KERSTEN ET AL: "Glycogenomics as a mass spectrometry-guided genome-mining method for microbial glycosylated molecules", PROCEEDINGS OF THE NATIONAL ACADEMY OF SCIENCES, vol. 110, no. 47, 4 November 2013 (2013-11-04), pages E4407-E4416, XP055270720, US ISSN: 0027-8424, DOI: 10.1073/pnas.1315492110
- GOMI S ET AL: "SF2446, new benzo[a]naphthacene quinone antibiotics. II. The structural elucidation", THE JOURNAL OF ANTIBIOTICS, NATURE PUBLISHING GROUP, GB, vol. 41, no. 4, 1 April 1988 (1988-04-01), pages 425-432, XP002723953, ISSN: 0021-8820, DOI: 10.7164/ANTIBIOTICS.41.425
- U. TAKEDA ET AL: "SF2446. New Benzo[a]naphthacene Quinone Antibiotics. I. Taxonomy and fermentation of the producing strain, isolation and characterization of antibiotics", THE JOURNAL OF ANTIBIOTICS, vol. 41, no. 4, 1 January 1988 (1988-01-01), pages 417-424, XP055082221,
- KAVITA TIWARI ET AL: "Rare actinomycetes: a potential storehouse for novel antibiotics", CRC CRITICAL REVIEWS IN BIOTECHNOLOGY, vol. 32, no. 2, 27 May 2011 (2011-05-27), pages 108-132, XP055270919, US ISSN: 0738-8551, DOI: 10.3109/07388551.2011.562482

## Description

### BACKGROUND OF THE INVENTION

Bacteria non-responsive to the activity of an antibiotic drug have been observed with increasing frequency in the past decades. The misuse and abuse of antibiotics has contributed to the appearance of resistant strains consequently causing a so-called "antibiotic resistance". The most recent World Economic Forum Global Risks reports have listed antibiotic resistance as one of the greatest threats to human health. Within the past twenty years the emergence of dangerous, resistant strains occurred with a worrying regularity. Moreover the incidence of antimicrobial resistance and its association with severe infections has increased reaching alarming proportions [Fair R.J., Tor Y.; "Antibiotics and Bacterial Resistance in the 21st Century" Perspect. Medicin. Chem. 6: 25-64 (2014)]. The Centers for Disease Control and Prevention (CDC) highlighted that all significant bacterial infections in the world could develop resistance to the antibiotic treatment of choice. The CDC estimates that, each year, nearly 2 million people in the United States acquire an infection while in a hospital, resulting in 90,000 deaths [Klevens R.M., Edwards J.R., Richards C. et al, "Estimating health care-associated infections and deaths in U.S. hospitals, 2002", Public Health Reports, 122: 160-166, (2007). World Health Organization. Antimicrobial Resistance: Global Report on Surveillance http://www.who.int/ drugresistance/ documents/surveillancereport/en/(2014)]. More than 70 percent of the bacteria that cause these hospital infections are resistant to at least one of the antibiotics commonly used to treat them. Recently, WHO (WHO/HSE/PED/AIP/2014.2) stated that new resistance mechanisms arise and diffuse globally threatening our ability to cure common infectious diseases, causing death and disability of individuals who until recently could conduct a normal course of life. In the 111th Congress, the Generating Antibiotic Incentives Now (GAIN) Act and the Strategies to Address Antimicrobial Resistance (STAAR) Act are introduced [Spellberg B., Blaser M., Guidos R.J., Boucher H.W. et al., "Combating antimicrobial resistance: policy recommendations to save lives", Clin Infect Dis.52 Suppl 5:S397-428 (2011)]. WHO stated that we are entering a 'post-antibiotic' era. The European Centre for Disease Prevention and Control (ECDC) evaluated that in the EU, Iceland, and Norway about 37,000 patients die as a consequence of a hospital-acquired infection each year; an additional 111,000 die as an indirect result of hospital-acquired infection [European Centre for Disease Prevention and Control (ECDC), Annual Epidemiological Report on Communicable Diseases in Europe, European Centre for Disease Prevention and Control, Stockholm, Sweden, (2008)] and around 25,000 patients die from a multidrug-resistant bacterial infection. Currently, the most common multidrug resistant (MDR) bacteria are *Enterococcus faecium, Staphylococcus aureus, Klebsiella pneumoniae, Acinetobacter baumannii, Pseudomonas aeruginosa,* and *Enterobacter spp.* which are collectively indicated as "ESKAPE" [Rice L.B., "Federal funding for the study of antimicrobial resistance in nosocomial pathogens: No ESKAPE", Journal of Infectious Diseases, 197, 8: 1079-1081 (2008)], with numerous studies adding *Clostridium difficile* or other Enterobacteriaceae [Peterson L.R., "Bad bugs, no drugs: no ESCAPE revisited", Clinical Infectious Diseases, 49, 6: 992-993(2009); Boucher H.W., Talbot G.H., Bradley J.S., Edwards J.E. et al.,"Bad bugs, no drugs: no ESKAPE! An update from the Infectious Diseases Society of America", Clin Infect Dis.48(1):1-12 (2009)]. Gram-positive pathogens, such as Staphylococcus, Streptococcus, Enterococcus, and Clostridium, represent a large proportion of serious infections worldwide. Several studies show that methicillin is no more a potent weapon against *Staphylococcus aureus.* Infections by MRSA strains resistant to glycopeptides, daptomycin, or linezolid (common anti-MRSA drugs) and by vancomycin resistant (VRE) strains resistant to daptomycin or linezolid (common anti-VRE drugs) are increasingly being reported, including reports of a transferable resistance mechanism to these drugs among staphylococci and enterococci.

Antibiotic resistance is spreading faster than the introduction of new compounds into clinical practice. To keep our current level of therapeutic efficacy, new antibiotics with new mechanisms of action and structure need to be developed. [Bassetti M., Righi E., "Development of novel antibacterial drugs to combat multiple resistant organisms", Langenbecks Arch Surg. 400(2):153-65 (2015)].

Natural products have proven to be the most fruitful source of leads for the development of drugs. It has been assessed that ninety five percent of the antibiotics described to date originate from leads discovered by screening natural product extracts or fractions. Many marketed antibacterial drugs are semisynthetic congeners of natural products, and are obtained from the chemical modification of fermentation products (e.g. rifampicin, cephalosporin, oritavancin, dalbavancin). Discovering novel natural products with broad-spectrum activity has become difficult. No really new class of broad-spectrum antibiotics has been developed since the quinolones were introduced to the clinic in 1962 and the oxazolidinone linezolid in 2000.

The present invention is directed to overcoming some of these deficiencies in the prior art. The prior art discloses several benzonaphtacene derivatives for treating bacterial infections (see US 2012/010163, Ratnakar N Askolar et al. 2009, JPH0196189 A, Anastasija Reimer et al. 2015, Kersten et al. 2013, Gomi S et al. 1988). The compounds of the present invention are very different from the compounds of the prior art in their structure and show improved antibiotic efficacy.

The present invention relates to novel compounds endowed with potent activity against microbial pathogens, the process for the preparation thereof, and the use thereof. These and other aspects and advantages therefrom will be better understood from the following description.

### DESCRIPTION OF THE INVENTION

The present invention concerns an antibiotic substance of microbial origin, arbitrarily denominated antibiotic FIIRV 104/18, which is a complex comprising factors F793, F795, F797 and F813, of formula (I) 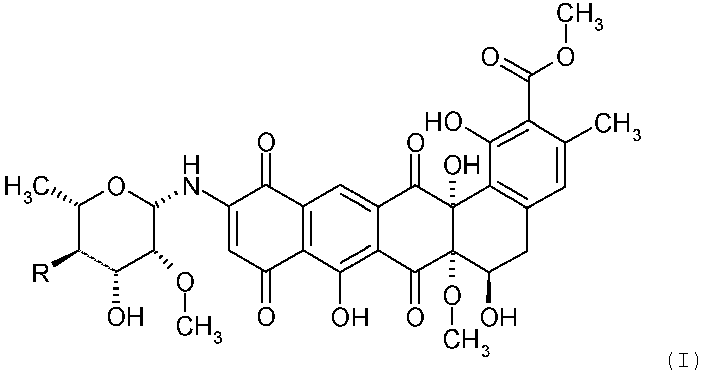 wherein R respectively represents

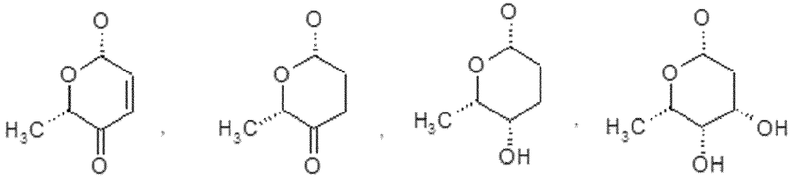

The invention includes also the isolated individual factors of the antibiotic FIIRV 104/18 complex and any mixture of two or more of said factors in any proportion.

Another object of the invention comprises a process for the preparation of antibiotic FIIRV 104/18 complex and the separation of the individual factors.

Additional object of the invention includes the use of antibiotic FIIRV 104/18 complex and the individual factors thereof as medicament.

Another object of the present invention is the micro-organism *Dactylosporangium* FIIRV sp. 104/18 deposited as number DSM 32068 on June 9, 2015 in the Deutsche Sammlung von Mikroorganismen und Zellkulturen GmbH, (DSMZ) Inhoffenstraße 7B, 38124 Braunschweig, Deutschland under Budapest Treaty. Further object of the invention comprises the use of biologically pure culture of the strain *Dactylosporangium* FIIRV sp. 104/18 DSM 32068, or a variant or mutant thereof maintaining the ability to produce the antibiotic FIIRV 104/18 complex and the individual factors when cultivated under submerged aerobic conditions in the presence of usable sources of carbon, nitrogen and inorganic salts.

The compounds of formula (I) are new glycosylated natural products (GNPs). A GNP consists of an aglycone and one or multiple glycosyl units [Thibodeaux C.J., Melançon C.E., Liu H.W., "Natural product sugar biosynthesis and enzymatic glycodiversification", Angew. Chem. Int. Ed. Engl. 47(51):9814-9859 (2008)], which often directly mediate the bioactivity of the compound [La Ferla B., Airoldi C., Zona C., et al., "Natural glycoconjugates with antitumor activity", Nat. Prod. Rep 28(3):630-648(2011)]. GNPs of microbial origin include many compounds with therapeutic applications, such as the antibiotic erythromycin [Staunton J., Weissman K.J. "Polyketide biosynthesis: A millennium review", Nat. Prod. Rep. 18(4):380-416 (2001)] and the insecticide avermectin [Ikeda H., Nonomiya T., Usami M., Ohta T., et al.,"Organization of the biosynthetic gene cluster for the polyketide anthelmintic macrolide avermectin in Streptomyces avermitilis", Proc. Natl. Acad. Sci. USA 96(17):9509-9514 (1999)]. GNPs represent a structurally very diversified class of natural products in terms of both the aglycone, i.e. the nonsugar portion of the molecule, and the glycosyl groups. GNPs are found in almost all major biosynthetic classes of natural products e.g. nonribosomal [Hubbard B.K., Walsh C.T., "Vancomycin assembly: Nature's way", Angew. Chem. Int. Ed. Engl. 42(7):730-765(2003)] and ribosomal peptides [Ding Y., et al., "Moving posttranslational modifications forward to biosynthesize the glycosylated thiopeptide nocathiacin I in Nocardia sp. ATCC 202099", Mol. Biosyst. 6(7):1180-1185 (2010)], polyketides [Ahlert J., et al., "The calicheamicin gene cluster and its iterative type I enediyne PKS", Science 297(5584):1173-1176 (2002)], terpenes [Gebhardt K, et al., "Phenalinolactones A-D, terpenoglycoside antibiotics from Streptomyces sp. Tü 6071", J. Antibiotics 64(3):229-232 (2011)] and alkaloids [Pathirana C., Jensen P.R., Dwight R., Fenical W., "Rare phenazine L-quinovose esters from a marine actinomycete", J. Org. Chem. 57:740-742 (1992)].

The new GNP compounds of formula (I) are characterized by the presence of a benzonaphthacene quinone group and a carbohydrate moiety.

In the carbohydrate moiety of each of the factors F793, F795, F797 and F813, the symbol R in the formula actually represents either a glycosidic radical deriving from respectively the following sugars: L-aculose, L-cinerulose A, L-rhodinose or 2-deoxy-L-fucose.

The Experimental Section of the present specification provides comparative tests carried out to verify the microbiological selectivity and efficacy of the compounds of formula (I) compared with known reference compounds and with comparative compound F683, which is a compound of formula (I) wherein R is OH.

Experimental tests carried out have shown that such compounds inhibit the growth of Gram-positive susceptible, resistant and multi-resistant pathogenic bacteria. Representative compounds F795 and F797 inhibit the growth of Gram-positive susceptible, resistant and multi-resistant pathogenic bacteria at concentrations in the range from 0.002 to 0.25 pg/mL. Experimental tests have shown that representative compound F797 inhibits replication of HeLa cells with an IC50 of 5 pg/mL. This suggests moderate cytotoxicity.

Other experimental tests have shown that factor F795 of antibiotic FIIRV 104/18 can treat an infection by *Staphylococcus aureus* in a murine model with an ED₅₀ of 0.80 mg/kg.

Antibiotic FIIRV 104/18 complex and the said isolated individual factors thereof are advantageously employed as antimicrobial agents against Gram positive bacteria, such as *Staphylococcus sp, Enterococcus sp, Streptococcus* sp, including MDR strains, in infectious diseases and against some Gram negative bacteria, such as *Moraxella sp.*

Accordingly, a further embodiment of the present invention relates to compounds of formula (I) for use as a medicament. In particular the present invention relates to compounds of formula (I) for their use in the treatment of infectious diseases, such as bacterial infectious diseases. In particular, according to the present invention, the antibiotic FIIRV 104/18 compounds of formula (I) are used in the treatment of bacterial infectious diseases caused by Gram-positive bacteria, including infectious diseases caused by multi-resistant bacteria as defined in this specification and claims. The compounds of the invention are generally active at doses in the range from 0.61 to 1.05 mg per kg of body weight in the animal model.

Antibiotic FIIRV 104/18 is a complex of novel antimicrobial agents presenting in their chemical structure a benzonaphthacene quinone moiety and N-linked 2-O-methyl-L-rhamnose. Several described benzonaphthacenes antibiotics have been obtained both from microbial sources as *Streptomyces* SF2446 [JP 01096189A and JP 63154695A; Takeda U., et al. "SF2446, New Benzo[a]naphthacene Quinone Antibiotics", J. Antibiotics 41(4):417-4224 (1988); Gomi S.,et al., "SF2446, New Benzo[a]naphthacene Quinone Antibiotics. II. The Structural Elucidation", J. Antibiotics 41 (4) :425-432 (1988)] and from chemical modification of the microbial product of *Streptomyces* AMRI-7957 [US 8754054 B2] Arenimycin A [Asolkar R.N., et al.,"Arenimycin, An Antibiotic Effective Against Rifampin- and Methicillin-Resistant Staphylococcus aureus from the Marine Actinomycete Salinispora arenicola", J. Antibiot (Tokyo) 63(1) :1-10 (2010)] and Arenimycin B, discovered using a glycogenomic approach [Kersten R.D., Ziemert N., Gonzalez D.J., et al., "Glycogenomics as a mass spectrometry-guided genome-mining method for microbial glycosylated molecules", Proc. Natl. Acad. Sci. USA 110(47): E4407-16 (2013)]. The benzonaphthacene quinones class produced by microbes comprise many compounds with therapeutic applications, such as the benanomycins and pradimycins [Oki T., Konishi M., Tomatsu K., et al., "Pradimicin, a novel class of potent antifungal antibiotics", J. Antibiotics 41:1701-1704 (1998); Shahzad-ul-Hussan S., Ghirlando R., Dogo-Isonagie C.I., Igarashi Y., et al., "Characterization and Carbohydrate Specificity of Pradimicin S", J. Am. Chem. Soc. 134(30):12346-12349 (2012)]. The physico-chemical data reported in the above-identified documents (e.g. mass spectroscopy data, molecular weight, content of carbohydrates) clearly show that the antibiotic FIIRV 104/18 complex as well as its components factors F793, F795, F797 and F813 are chemical entities distinct from those described in the prior art. Antibiotic FIIRV 104/18 factors are di-glycosylated benzonaphthacene quinones whose first sugar moiety is a N-linked 2-O-methyl-L-rhamnose. The SF2446 microbial compounds, the AMRI-7957 microbial compound and all its chemically derived products are monoglycosilated with a N-linked 2,4-di-O-methyl-rhamnose. Arenimycin B is a diglycosylated benzonaphthacene quinone, whose second carbohydrate moiety, D-forosamine, is different from those present in the antibiotic FIIRV 104/18 factors; moreover the benzonaphthacene quinone core is differently oxidized: it has a H instead of OH in position 6. Arenimycins share the benzo[a]naphthacene quinone core with Pradimicins, but have different oxidation patterns and different glycosylation sites and groups. According to the above mentioned prior art the benzo[a]naphthacene quinone skeleton with no carbohydrate moiety is observed in few compounds: collinone, ansacarbomitocins, G-2N, G-2A,KS-61910 and BE-19412A.

The present invention relates also to pharmaceutical compositions comprising the compounds of formula (I). According to this invention the pharmaceutical composition for use as medicament for the treatment of infectious diseases contain the antibiotic FIIRV 104/18 complex, an isolated individual factor thereof or a combination of one or more of said factors in any proportion.

The compounds of the present invention, in their pharmaceutically acceptable form, may be administered via oral, topical or parenteral route depending on the treatment to be performed. These compounds can be formulated into different dosage forms according to the route of administration. The preparations for oral administration may be in the form of tablets, capsules, lozenges, liquid solutions or suspensions. As known in the art, tablets, capsules and lozenges may contain usual excipients in addition to the active ingredient, for example extenders such as lactose, calcium phosphate, sorbitol and the like; lubricants such as magnesium stearate, polyethylene glycol (PEG), binding agents such as polyvinyl pyrrolidone, gelatine, sorbitol, acacia, flavoring agents, disintegrating agents and dispersing agents.

Liquid preparations, generally in the form of aqueous or oily solutions or suspensions, may contain conventional additives such as dispersing agents. For topical use, the compounds of formula (I) of the invention can also be prepared in suitable forms to be absorbed by either the mucous membranes of nose and throat or bronchial tissues, and they may advantageously be in the form of a spray. Topical applications can be formulated as ointments, lotions, gels or powders in hydrophobic or hydrophilic bases.

A further aspect of this invention consists in providing the antibiotic complex or isolated individual factor(s) thereof, for use as medicament for for the treatment of bacterial infection, in particular a bacterial infection caused by bacteria of it least one of the genera Enterococci, Streptococci, Staphylococci and Moraxella, comprising administering an effective amount of antibiotic FIIRV 104/18 complex or an individual factor thereof as defined above to a patient in need thereof.

### PROCESS

According to a preferred aspect of the invention, the process for the preparation of compounds of formula (I) comprises cultivating *Dactylosporangium* FIIRV sp. 104/18 deposited with number DSM 32068 on June 9, 2015 in the Deutsche Sammlung von Mikroorganismen und Zellkulturen GmbH under the Budapest Treaty or a variant or mutant thereof capable to produce a compound of formula (I), collecting the product of formula (I) from the mycelium and/or fermentation broths, isolating the pure compound of formula (I) by chromatographic techniques.

### PRODUCTION STRAIN AND FERMENTATION

The production of the compounds of formula (I) is obtained by cultivating a strain of *Dactylosporangium* capable of producing the product of formula (I), such as *Dactylosporangium* FIIRV sp. 104/18 DSM 32068 or a variant or mutant thereof which maintains the ability to produce the compound of formula (I). In a preferred aspect, the production of the compounds of formula (I) is obtained under aerobic conditions in an aqueous production medium containing easily digestible and usable sources of carbon, nitrogen and inorganic salts, such as starch, dextrin, glucose, maltose and the like as the carbon source, soybean meal, peptone, meat extract, casein hydrolyzate, tryptone, yeast extract and the like as the nitrogen source. The medium can be supplemented with salts capable of providing sodium, potassium, iron, zinc, magnesium, calcium, ammonium, chloride, carbonate, sulphate, phosphate, nitrate and the like ions.

The production strain for the compounds of formula (I) is preferably grown in a flask or small fermenter, and the culture is used to inoculate fermentation reactors for the production. The pre-culture medium can be the same or different from that used for the production on an increased scale. According to a preferred aspect, *Dactylosporangium* FIIRV sp. 104/18 DSM 32068 is grown on ISP3 agar plates (see Experimental Section) in which the strain forms colonies developing orange vegetative mycelium and globose bodies. The growth temperature for the strain *Dactylosporangium* FIIRV sp. 104/18 DSM 32068 is 24-40°C, preferably 28-32°C. During fermentation, the production of the compounds of formula (I) is monitored by HPLC, and it generally occurs within 11-14 days of fermentation.

### MORPHOLOGICAL AND CULTURAL CHARACTERISTICS OF Dactylosporangium FIIRV sp 104/18. DSM 32068

*Dactylosporangium* FIIRV sp. 104/18 is isolated in the soil environment and deposited on 9 June, 2015 with the DSMZ under the provision of the Budapest Treaty. The strain is accorded accession number DSM 32068.

Morphological observation of the strain *Dactylosporangium* FIIRV sp. 104/18 DSM 32068 is made on cultures grown at 28°C for 15 days using an optic microscope with 40X long-working distance objective. Abundant growth and production of light orange-brown vegetative mycelium are observed on yeast extract and malt extract agar (ISP medium 2) and on inorganic salts-starch agar (ISP medium 4), oatmeal agar (ISP medium 3) and on peptone-yeast extract-iron agar (ISP medium 6). Poor growth is observed on glycerol asparagine agar (ISP medium 5) and on tyrosine agar (ISP medium 7). Aerial hyphae are absent in all media tested. A brown diffusible pigment is released in ISP3 agar medium with ageing of the culture. Globose bodies are produced abundantly on ISP 2 and 4 but moderate production is observed on ISP3 and humic acid-vitamin agar.

The temperature for growing strain *Dactylosporangium FIIRV* sp. 104/18 DSM 32068 is 24-40°C, preferably 28-32°C; no growth occurs below 15°C and above 40°C.

The growth is totally inhibited in the presence of more than 1% (w/v) NaCl and the pH range for growth is 5.5-8.5, with an optimum at 7.0.

### 16S rRNA GENE SEQUENCE OF Dactylosporangium FIIRV sp. 104/18 DSM 32068

SEQ ID NO *1* shows the partial sequence, consisting of 1294 nucleotides, of the gene encoding the 16S rRNA of the strain *Dactylosporangium* FIIRV sp. 104/18 DSM 32068. This sequence is compared with those deposited in public databases, and it is found to be highly related (>99%) to the 16S rRNA sequence of various strains of *Dactylosporangium.*

As with other micro-organisms, the characteristics of the production strain for the compound of formula (I) can be mutated. For example, artificial variants and mutants of the strain can be obtained by treatment with known mutagenic agents such as UV rays, chemicals such as nitrous acid, N-methyl-N-nitrosoguanidine and others.

### EXTRACTION AND PURIFICATION OF ANTIBIOTIC FIIRV 104/18 COMPLEX

As mentioned above, antibiotic FIIRV 104/18 complex is found both in the mycelium (80% or predominantly) and in the filtered or centrifuged fraction of the fermentation broth. The harvested broth may be processed to separate the mycelium from the supernatant of the fermentation broth and the mycelium may be extracted with a water-miscible solvent to obtain a solution containing the antibiotic FIIRV 104/18 complex, after removal of the spent mycelium. This mycelium extract may then be processed separately or in pool with the supernatant according to the procedures reported hereafter for the supernatant fraction. For maximum recovery of the product, these two fractions are processed separately in the primary recovery steps, as exemplified below.

When the water-miscible solvent may cause interferences with the operations for recovering the antibiotic from the mycelium extract, the water-miscible solvent may be removed by distillation or may be diluted with water to a non-interfering concentration. The term "water-miscible solvent" as used in this application, is intended to have the meaning currently given in the art of this term and refers to solvents that, at the conditions of use, are miscible with water in a reasonably wide concentration range. Examples of water-miscible organic solvents that can be used in the extraction of the compounds of the invention are: methanol, ethanol, propanol, acetone, dioxane, and acetonitrile (CH₃CN).

The recovery of the antibiotic FIIRV 104/18 complex from the supernatant of the fermentation broth of the producing micro-organism is conducted by means of a technique selected from at least one of: extraction with water immiscible solvents, precipitation by adding non-solvents or by changing the pH of the solution, absorption chromatography, partition chromatography, reverse phase partition chromatography, ion exchange chromatography, molecular exclusion chromatography and the like or a combination of two or more of said techniques. A procedure for recovering the compounds of the invention from the filtered fermentation broth includes extraction of antibiotic FIIRV 104/18 complex with water-immiscible organic solvents, followed by precipitation from the concentrated extracts, possibly by adding a precipitating agent. Also in this case, the term "water-immiscible solvent" as used in this application, is intended to have the meaning currently given in the art to said term and refers to solvents that, at the conditions of use, are slightly miscible or practically immiscible with water in a reasonably wide concentration range, suitable for the intended use. Examples of water-immiscible organic solvents that can be used in the extraction of the compounds of the invention from the fermentation broth are: alkanols of at least four carbon atoms which may be linear, branched or cyclic such as n-butanol, 1-pentanol, 2-pentanol, 3-pentanol, 1-hexanol, 2-hexanol, 3-hexanol, 3,3-dimethyl-l-butanol, 4-methyl-l-pentanol, 3-methyl-l-pentanol, 2,2-dimethyl-3-pentanol, 2,4-dimethyl-3-pentanol, 4,4-dimethyl-2-pentanol, 5-methyl-2-hexanol, 1-heptanol, 2-heptanol, 5-methyl-1-hexanol, 2-ethyl-l-hexanol, 2-methyl-3-hexanol, 1-octanol, 2-octanol, cyclopentanol, 2-cyclopentylethanol, 3-cyclopenthyl-1-propanol,cyclohexanol, cycloheptanol, cyclooctanol, 2,3-di-methyl-cyclohexanol,4-ethylcyclohexanol, cyclooctylmethanol 1-nonanol, 2-nonanol, 1-decanol, 2-decanol, and 3-decanol; acetic acid esters such as ethyl acetate, isopropyl acetate, ketones of at least five carbon atoms such as methylisopropylketone, methylisobutylketone, methyl-n-amylketone, methylisoamylketone and mixtures thereof. As known in the art, product extraction from the filtered fermentation broth may be improved by adjusting the pH at an appropriate value, and/or by adding a proper organic salt forming an ion pair with the antibiotic, which is soluble in the extraction solvent. As known in the art, phase separation may be improved by salting the aqueous phase. When, following an extraction, an organic phase is recovered containing a substantial amount of water, it may be convenient to azeotropically distill water from it. Generally, this requires adding a solvent capable of forming minimum azeotropic mixtures with water, followed by the addition of a precipitating agent to precipitate the desired product, if necessary. Representative examples of organic solvents capable of forming minimum azeotropic mixtures with water are: n-butanol, benzene, toluene, butyl ether, carbon tetrachloride, chloroform, cyclohexane, 2,5-dimethylfuran, and m-xylene; the preferred solvent being n-butanol. Examples of precipitating agents are petroleum ether, lower alkyl ethers, such as ethyl ether, propyl ether, and butyl ether.

According to a preferred procedure for recovering antibiotic FIIRV 104/18 complex, the fermentation broth is first cooled to 20°C and then centrifuged to separate cells (mycelium) from supernatant. The supernatant can be contacted with an adsorption matrix followed by elution with a polar, water-miscible solvent or a mixture thereof, concentration to an oily residue under reduced pressure and precipitation with a precipitating agent of the type mentioned above. Examples of adsorption matrixes that can be conveniently used in the recovery of the compounds of the invention, are polystyrene or mixed polystyrene-divinylbenzene resins (e.g. M112 or S112, Dow Chemical Co.; Amberlite^{®} XAD2 or XAD4, Rohm & Haas; Diaion HP20, Mitsubishi), acrylic resins (e.g. XAD7 or XAD8, Rohm & Haas), polyamides such as polycaprolactams, nylons and cross-linked polyvinylpyrrolidones (e.g. Polyamide-CC 6, Polyamide-SC 6, Polyamide-CC 6.6, Polyamide-CC 6AC and Polyamide-SC 6AC, Macherey-Nagel & Co., Germany; PA 400, M.Woel AG, Germany), the polyvinylpyrrolidone resin PVP-CL, (Aldrich Chemie GmbH & Co., KG, Germany) and controlled pore cross-linked dextrans (e.g. Sephadex^{®} LH-20, Pharmacia Fine Chemicals, AB). Preferably, polystyrene resins are employed, particularly preferred being the Diaion HP20 resin. In the case of the use of polystyrene resins, polystyrene- divinylbenzene resins, polyamide resins or acrylic resins a preferred eluent is a water-miscible solvent or its aqueous mixtures. The aqueous mixtures can contain buffers at appropriate pH value. Also in this case, the term "water-miscible solvent", as used in this description and claims, is intended to have the meaning currently given in the art to said term as described above. The mycelium is treated with an organic solvent such as ethanol, methanol, acetone. A more detailed description of the methods for recovering the antibiotic FIIRV 104/18 complex from the fermentation batch is given herein below.

### ANTIBIOTIC FIIRV 104/18 COMPLEX RECOVERY FROM SUPERNATANT

According to a preferred procedure, hydrated DIAION HP-20 adsorbent resin is added to the supernatant (1g wet weight per 40 mL broth solution) and agitated for 2-16 hours during which time the produced factors are almost entirely bound to the resin. The resin is recovered by sieving (50 mesh). The resin is sequentially washed in a column format with 8 column volumes of water, 4 column volumes of 20% ethanol. The complex is then eluted in approximately 1-2 column volumes of either 1000 ethanol or methanol or 100% acetonitrile. Elution fractions containing the bulk of the product are pooled and evaporated under vacuum to minimal volume. This slurry is then suspended in water and extracted with water immiscible solvents, preferably n-butanol or ethyl acetate. The organic phase is concentrated and used for further purification steps.

### ANTIBIOTIC FIIRV 104/18 COMPLEX RECOVERY FROM MYCELIUM

To the fermentation cell solids recovered as described, a water miscible solvent such as ethanol is added and allowed to contact for 2 to 24 hours (2 mL ethanol/g mycelium). The suspension is centrifuged or filtered and the clear ethanol solution is concentrated to minimal volume under vacuum. The resulting slurry is then suspended in water and extracted with a water immiscible solvent, such as n-butanol or ethyl acetate. Alternatively, the same slurry is diluted with water up to 20% and loaded on Diaion HP-20 resin (1g of hydrated resin per 8 mL ethanol solution). The resin is recovered by sieving (50 mesh). The resin is sequentially washed in a column format with 8 column volumes of 20% ethanol; no or only minimal product losses occur in the washes. The product is then eluted in approximately 1-2 column volumes of either 100% ethanol or 100% acetonitrile. Elution fractions containing the bulk of the product are pooled and evaporated to under vacuum to minimal volume. This slurry is then suspended in water and extracted with water immiscible solvents, such as n-butanol or ethyl acetate.

Alternatively, the mycelium is extracted with a water immiscible solvent such as ethyl acetate and allowed to contact for 2 to 24 hours. The suspension is centrifuged or filtered, the resulting organic solution is washed with water, dried and evaporated under vacuum. The resulting solid is then subjected to further purification steps.

### ANTIBIOTIC FIIRV 104/18 COMPLEX RECOVERY FROM WHOLE MICROBIAL CULTURE

According to this method the isolation of the antibiotic FIIRV 104/18 complex is carried out on the whole microbial culture by partition chromatography, followed by extraction with a water-miscible solvent. Preferentially, an adsorbing resin such as hydrated DIAION HP-20 resin is added to the strain culture (1g wet weight per 40 mL) and agitated for 2-16 hours during which time the product is almost entirely bound to the resin. Then, the strain culture can be filtered or centrifuged and resin plus mycelium are contacted with a water miscible solvent such as ethanol for 2 to 24 hours (2 mL ethanol/g mycelium) as described above. The clear solution obtained is concentrated to minimal volume under vacuum, suspended in water and extracted with water immiscible solvents, such as ethyl acetate. The organic phase is concentrated and used for further purification steps.

### ANTIBIOTIC FIIRV 104/18 COMPLEX PURIFICATION

The following procedures for the isolation and purification of the antibiotic complex may be carried out on the pooled extracts from the broth supernatant or from the mycelium. For example, when the portion of the antibiotic product contained in the centrifuged or filtered fermentation broth or supernatant is recovered by absorption on an absorption resin and the portion of the antibiotic product contained in the mycelium is extracted therefrom with a water-miscible solvent, followed by adsorption onto an absorption resin, the eluted fractions from each of the two sets of absorption resins may be combined, optionally after concentration, and then further processed as a unitary crop. Alternatively, when the two sets of absorption resins utilized for the independent extraction stages are of the same type and have the same functional characteristics, they may be pooled together and the mixture may be submitted to a unitary elution step, for instance, with a water-miscible solvent or a mixture thereof with water. In any case, whatever may be the procedure adopted for recovering the crude antibiotic FIIRV 104/18 complex, the successive purification step is usually carried out on the mixture of the crude materials resulting from the combination of the products originating from the separate extraction stages. Purification of the crude antibiotic FIIRV 104/18 complex is preferably conducted by means of chromatographic procedures. Examples of these chromatographic procedures are those reported in relation to the recovery step and include also chromatography on stationary phases such as silica gel, alumina, or reverse phase chromatography on silanized silica gel having various functional derivatizations, and eluting with water miscible solvents or aqueous mixture of water-miscible solvents of the kind mentioned above. For instance, preparative HPLC chromatography may be employed, using reverse phase C8 or C18 as stationary phase and a mixture of HCOONH₄ buffer: CH₃CN as eluting system. The active fractions recovered from the purification step are pooled together, concentrated under vacuum, precipitated by addition of a precipitating agent of the kind mentioned above and dried or lyophilized in individual or iterative rounds. In the case the product contains residual amounts of ammonium formate or other buffering salts, these may be removed by absorption of the antibiotic FIIRV 104/18 complex on solid phase extraction column, for instance a reverse phase resin column such as SPE Isolute C18 StepBio (STEPBIO s.r.l., Bologna-Italy) followed by washing with distilled water and elution with an appropriate aqueous solvent mixture, e.g. methanol/water. The antibiotic complex is then recovered by removing the elution solvents. Accordingly, a purified antibiotic FIIRV 104/18 complex dried preparation is obtained as a red powder. As usual in this art, the production as well as the recovery and purification steps may be monitored by a variety of analytical procedures including inhibitory assay against a susceptible micro-organism and analytical control using the HPLC or HPLC coupled with mass spectrometry. A preferred analytical HPLC technique is performed on a HPLC system Accela Instrument (Thermo Fisher Scientific, San Jose, CA) equipped with a chromatographic column Phenomenex Luna C18 5µ (250 × 4.6 mm) eluted at 1 mL/min flow rate and at room temperature. Elution is with a multistep program: Time=0 (40% phase B); Time=4 min (40% Phase B); Time=16 min (55 % of phase B); Time=18.5 min (90% of phase B). Phase A is CH₃CN:10 mM ammonium formate buffer (pH 4.5) 5:95 (v/v) and Phase B is CH₃CN. A Photodiode Array (PDA) detector is used. The effluent from the column is splitted in a ratio 5:95 and the majority (ca. 950 µl/min) is diverted to PDA detector. The remaining 50 µl/min are diverted to the electrospray ionization (ESI) interface of a LTQ-xl ion trap mass spectrometer (Thermo Fisher Scientific, San Jose, CA). The mass spectrometric analysis is performed under the following conditions:
Sample inlet conditions: sheath gas (N₂) 45 psi; auxiliary (aux) gas (N₂) 35 psi; capillary heater 275°C; sample inlet voltage settings: polarity both positive and negative; ion spray voltage +/- 3.5 kV; capillary voltage +/- 175V; scan conditions: maximum ion time 200 ms; ion time 5 ms; full micro scan 3; segment: duration 30 min, scan events positive (150-2000 m/z) and negative (150-2000 m/z).

According to the above analytical HPLC conditions the antibiotic FIIRV 104/18 factors show retention times (rt) of 5.7 min for F683 and F813, 12.2 min for F797, 15.4 min for F795, and 16.3 min for F793.

### INDIVIDUAL FACTORS PURIFICATION

The five factors of antibiotic FIIRV 104/18 complex can be separated from a purified sample of antibiotic FIIRV 104/18 complex by means of preparative HPLC. Factors are separated and purified on a Phenomenex Luna Preparative C18 column 5µ (250 × 21.2 mm) (Phenomenex, Torrance CA) from the purified antibiotic FIIRV 104/18 complex dissolved in DMSO:H₂O:CH₃CN 1:1:1 (v/v) using a 35 minutes stepwise gradient elution (including periods of isocratic elution) from 35% to 55% of phase B at 20 mL flow rate. Phase B is CH₃CN. Phase A is ammonium formate 20 mM pH4.5. Factors F683 and F813 co-elute with 40% of phase B at rt of 13.0 min, pure factor F797 elutes with 42% phase B at rt 17.5 min, factor F795 elutes with 48% phase B at 27.5 min and factor F793 elutes with 50% phase B at 30.5 min. The eluted fractions containing pure antibiotic FIIRV 104/18 factor F797, F795 and F793 are collected and concentrated to aqueous phase under vacuum. The water solutions are extracted with ethyl acetate or n-butanol and the organic layers are concentrated to minimal volume; then petroleum ether or hexane or diethyl ether are added. Pure factors are obtained by precipitation and recovered by filtration. Alternatively the water solutions can be lyophilized.

Factors F683 and F813 are separated and purified from the above reported mixed fractions by preparative HPLC using a Phenomenex Luna Phenyl Hexyl 5µ (250 × 10 mm) column. A sample of antibiotic FIIRV 104/18 factor F683 and F813 mixture is dissolved in DMSO:H₂O:CH₃CN 1:1:1 (v/v) and a 25 minutes linear gradient elution from 25% to 40% of phase B at 8 mL flow rate is performed. Phase B is CH₃CN. Phase A is ammonium formate 20 mM pH4.5. Pure factor F683 elutes at 16.5 min rt with 36% phase B, and pure factor F813 elutes at 17.5 min rt with 37% phase B. Each group of eluted fractions containing pure antibiotic FIIRV 104/18 factor F683 and factor F813 is collected and concentrated to aqueous phase under vacuum. The water solutions are extracted with ethyl acetate or n-butanol and the organic layers are concentrated to minimal volume; then petroleum ether or hexane or diethyl ether are added. Pure factors are obtained by precipitation and recovered by filtration. Alternatively the water solutions can be lyophilized.

### PHYSICO-CHEMICAL CHARACTERISTICS OF FACTOR F795 OF ANTIBIOTIC FIIRV 104/18 COMPLEX

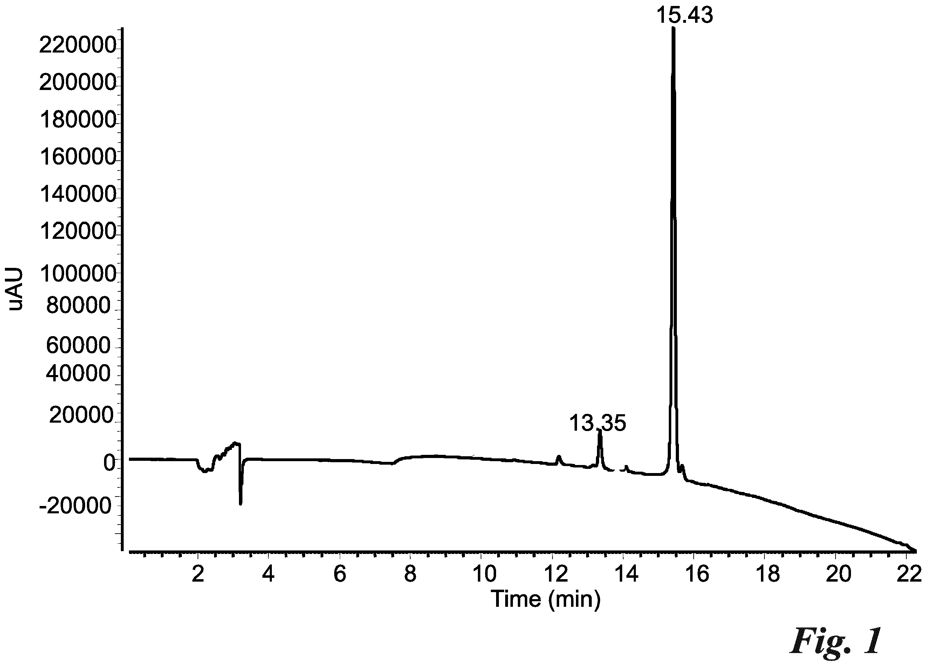

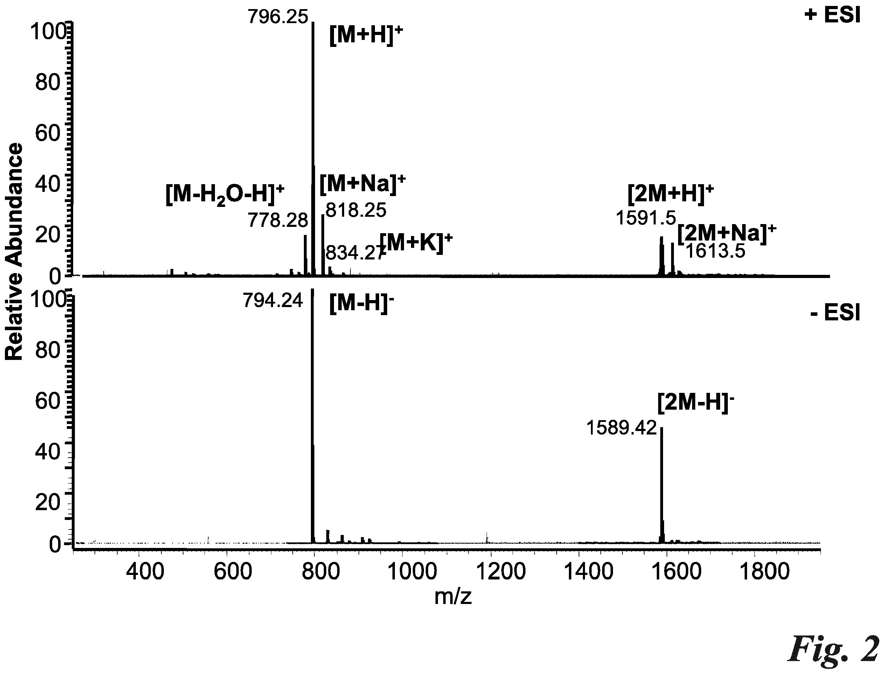

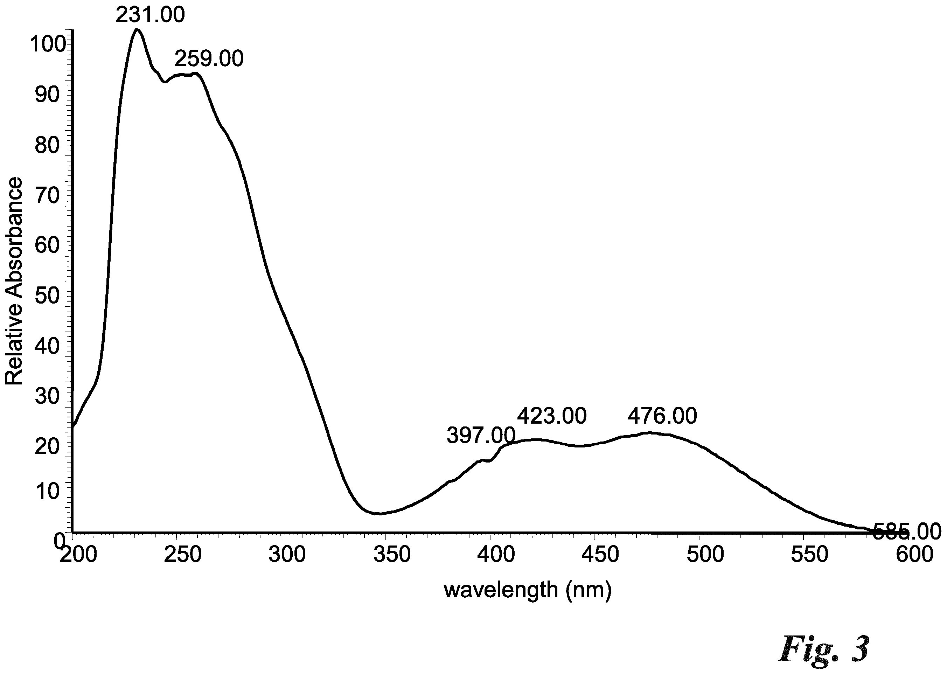

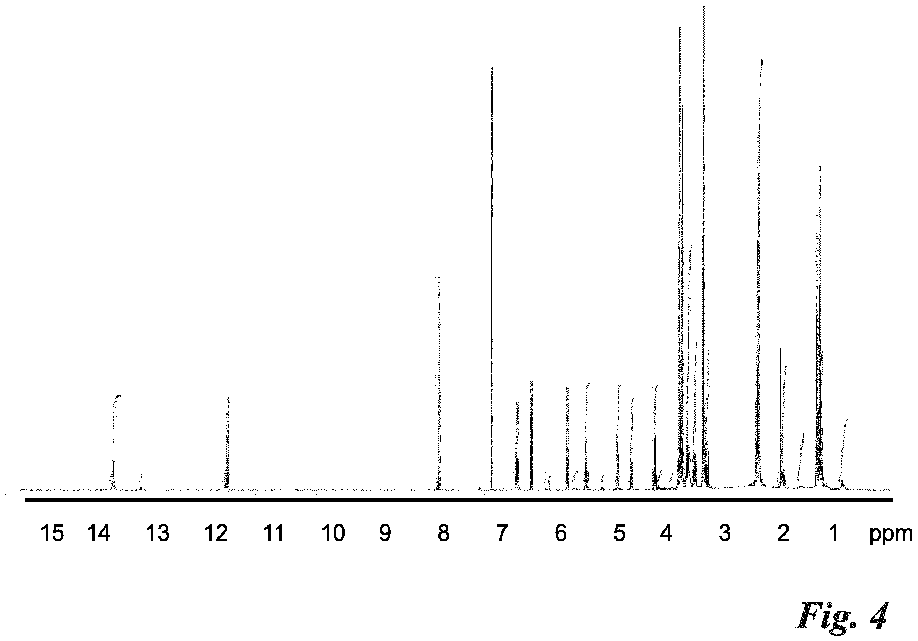

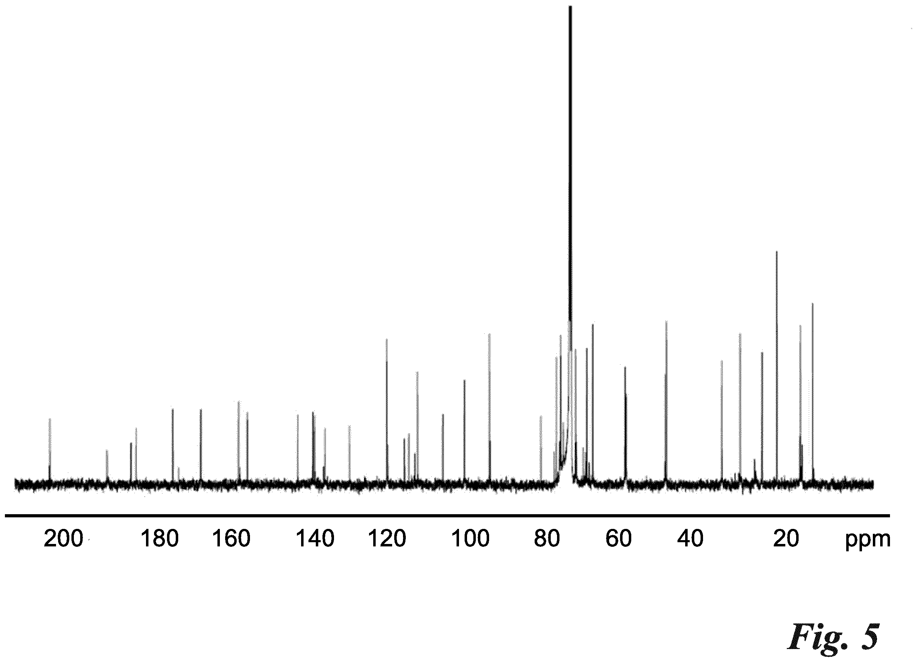

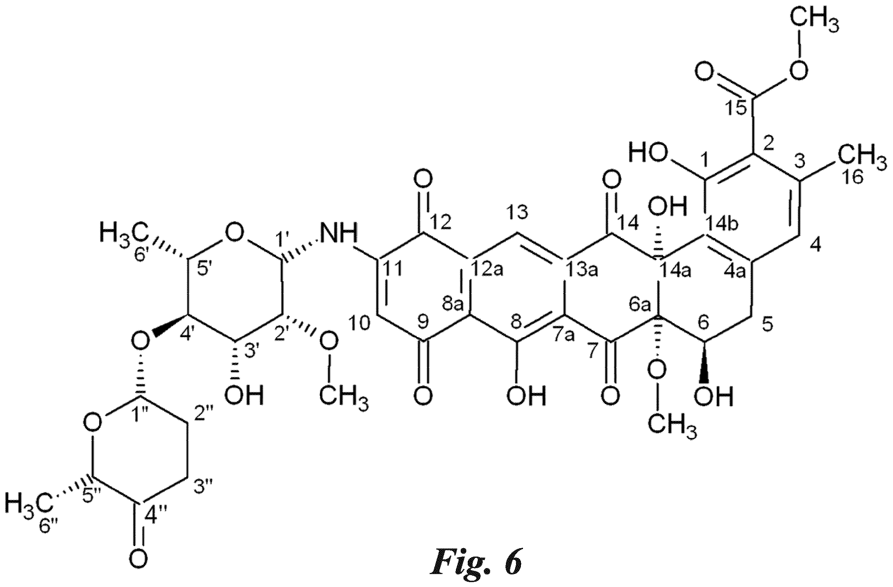

F795 has a molecular weight of 795. The HPLC-MS analysis is shown in Figure 1.
A) Mass spectrometry: in MS experiments on a Thermo Scientific LTQ-xl instrument (Thermo Fisher Scientific, San Jose, CA) fitted with an electrospray source, using Thermo Fisher Scientific calibration mix, F795 shows a mono-charged ion at m/z 796.25 corresponding to [M+H]⁺, a mono-charged ion at m/z 818.25 corresponding to [M+Na]⁺, a mono-charged ion at m/z 834.27 corresponding to [M+K]⁺, a double-charged ion at m/z 1591.5 corresponding to [2M+H]⁺ and a double-charged ion at m/z 1613.5 corresponding to [2M+Na]⁺. In negative mode F795 gives a mono charged ion at m/z 794.24 corresponding to [M-H]⁻ and a double-charged ion at m/z 1589.42 corresponding to [2M-H]⁻. In positive mode, the electrospray conditions are the following: spray voltage 2.7 kV; capillary temperature 275°C; capillary voltage 4.5 V; spectra can be obtained during a HPLC analysis or in infusion mode at 10 µl/min. Spectra are recorded from a 0.1 mg/mL solution in CH₃CN/H₂O ratio 60/40 (v/v) and are reported in Figure 2 (full-scan low resolution spectrum).
B) MS/MS analysis of the mono charged ion is performed with the observed main fragmentations: mono charged ions at m/z 778, 764, 746, 732, 714, 696, 666, 650, 634, 602, 524, 506, 474, 442 and 414. The pathway of fragmentation is similar to those observed for the other factors of the antibiotic FIIRV 104/18 complex. A neutral loss of 160 mass units is observed and suggests the presence of C₇H₁₂O₄ moiety. The SF2446 compounds [Gomi et al., "SF2446, New Benzo[a]naphthacene Quinone Antibiotics. II. The Structural Elucidation", J. Antibiotics 41 (4) :425-432 (1988)] and the compounds described by the patent US 8754054 B2 are characterized by the presence of a C₈H₁₄O₄ moiety.
C) The molecular weight of factor F795 is obtained from the analysis of high-resolution electrospray ionization acquired on an ESI-FT ICR SolariX (Bruker Daltonik, Germany, GmbH). When the electrospray interface is set in negative ionization mode, the conditions are: spray voltage +3200 V; cap exit -210.0 V. Nitrogen is used as a drying at 3.7 liters/min and nebulizing gas at 1.0 bar; the ion transfer capillary is kept at 180°C. When the electrospray interface is in positive ionization mode, the conditions are: spray voltage -4300 V; cap exit +210.0; drying gas at 4 liters/min; nebulizing gas at 1.3 bar; the ion transfer capillary is kept at 200°C. Mass signals are recorded in the m/z 150-3000 mass range. Accurate mass calibration is performed in the range m/z 250-1000 by infusing a solution of sodium trifluoroacetate at the appropriate concentration. Sample solutions are introduced in the electrospray source using a syringe pump operating at 4pl/min. Ions are observed at m/z 796.24576 [M+H]+ and 794.22955 [M-H]⁻, error <1.5ppm. In table 1 are reported the observed values measured in negative and positive mode. The deduced molecular formula is C₃₉H₄₁N₁O₁₇. The isotopic profile is coherent with the deduced molecular formula. Collision-induced dissociation(CID) fragmentation experiments are performed by evaluating different collision energy settings (from 15 to 30V). Mass spectra are elaborated using the DataAnalysis software (Bruker Daltonics, GmbH) or Xcalibur Thermo scientific. When submitted to higher energy, ions at m/z 442.05 and 524.12 are observed. When acquiring spectra at high energy the presence of a signal at 524.11767 is found, which corresponds to the aglycone moiety and has formula of C₂₆H₂₁N₁O₁₁.
D) The U.V. spectrum of factor F795, performed in 10 mM ammonium formate pH 4.5: CH₃CN (in ratio 50:50) with a Thermo Scientific Accela PDA detector (Thermo Fisher Scientific Inc. USA), during a HPLC analysis, shows absorption maxima at 216, 228, 251, 300, 415 and 475nm, which indicated the presence of a complex naphthoquinone moiety. UV-visible spectrum is reported in Figure 3.
E) Determination of glycosidic moiety. In tandem mass spectrometry (MS/MS) experiment at high resolution a neutral loss corresponding to 112.06808 occurs suggesting that the second sugar moiety is L-cinerulose A. Further loss of 160.7298 corresponding to the loss of first sugar moiety C₇H₁₂O₄. Moreover, an ion at m/z 524.11767 is detected which corresponds to the aglycone moiety. Its molecular formula is C₂₆H₂₁NO₁₁ (Table 1).
F) Factor F795 is hydrolyzed under acidic conditions (1 or 6N HCl at 100°C for 1-24 hours); the hydrolyzed mixture is analyzed and purified by HPLC chromatography. In this conditions the amino group is hydrolyzed and the generated aglycone moiety is C₂₆H₂₀O₁₂. Table 1 reports the accurate mass measured.
G) ¹H-, ¹³C- and 2D NMR experiments are recorded in CDCl₃ at 300°K on a Bruker Avance 500 MHz instrument. ¹H-NMR spectrum is reported in Figure 4. For the following discussion, reference is made to the overall structure formula represented in Figure 6, where the numbering of the carbon atoms is assigned in conformity with Arenimycin A [Asolkar R.N.,et. al"Arenimycin, An Antibiotic Effective Against Rifampin- and Methicillin-Resistant Staphylococcus aureus from the Marine Actinomycete Salinispora arenicola", J. Antibiotics 63(1):1-10 2010)] and Arenimycin B[Kersten R.D.,et al.,"Glycogenomics as a mass spectrometry-guided genome-mining method for microbial glycosylated molecules" Proc. Natl. Acad. Sci. USA, 110(47):E4407-16 (2013)]. According to integration, 6 methyl signals, 3 methylene and 11 methine protons are detected. In addition, two phenolic exchangeable protons H-1 and H-8 are shown. Most signals belonging to the main scaffold resemble typical chemical shifts of the known benzonaphthacene quinone molecules. The presence of a doublet at 4.98 ppm coupled with one of the adjacent methylene protons suggest the presence of a hydroxylic group in position 6. Full assignments are completed in agreement with multiplicity and coupling constants (Table 2). Overall stereostructure is assigned by Nuclear Overhauser Spectroscopy (NOESY) experiments; in particular, H-6 shows strong Nuclear Overhauser Effect (NOE)with 6a-OCH3, a strong NOE also with H-5A and a weaker NOE with H-5B, as expected. Compared to known structures, substantial differences are observed in the glycosydic region. The N-glycosyl sugar moiety of the structure of factor F795 is assigned by the analysis of combined one-dimensional (1D) and two-dimensional (2D) NMR data. The ¹H NMR spectrum of FACTOR F795 shows an anomeric proton signal at δ 4.74 (1H, d, J=9.0 Hz) and four -CH-OH signals between δ 3.83 and δ 3.41, which define a hexopyranosyl ring. ¹H-¹H-COSY (Correlation SpectroscopY) and Heteronuclear Multiple Bond Correlations (HMBC) are in agreement. The methoxyl group is assigned to position 2' and confirmed by NOE effect with H-6' and H-1": this clearly indicates that the sugar is L-2'-O-methyl-rhamnopyranose. Also, more HMBC and NOE correlations confirm the presence of a second sugar moiety, namely L-cinerulose A, bound to 2'-O-methyl-rhamnopyranose in position 4'. Moreover, a very characteristic quartet signal at 4.31 ppm assigned to position 5" clearly indicates the presence of an adjacent quaternary carbon, corresponding to position 4" carbonyl group. ¹³C-NMR reported in Figure 5 confirms this interpretation with a 4" carbonyl signal at 211 ppm. Also, the ¹³C-APT (Attached Proton Test) experiment shows 6 methyl, 3 methylene, 11 methine and 19 quaternary carbon peaks. Two-dimensional ¹H-¹³C-HSQC (Heteronuclear Single Quantum Coherence) cross peaks are in agreement with the above reported data. In conclusion, NMR shows all signals consistent with the molecular formula reported below and in Figure 6. 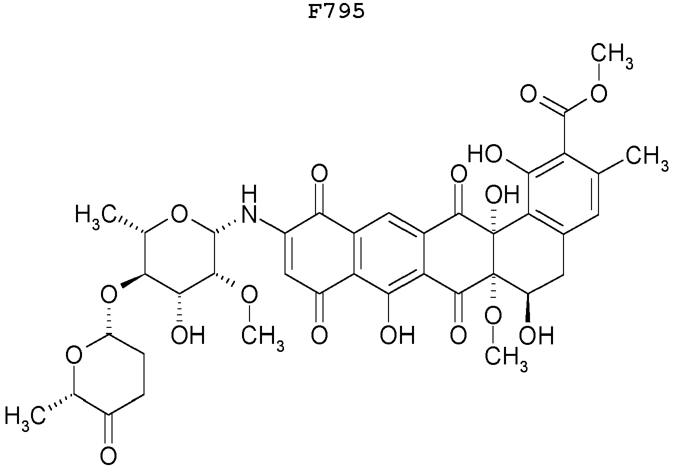 (6R,6aS,14aR)-methyl 11-[(2S,3R,4R,5R,6S)-4-hydroxy-3-methoxy-6-methyl-5-{[(2S,6S)-6-methyl-5-oxotetrahydro-2H-pyran-2-yl]oxy}tetrahydro-2H-pyran-2-ylamino]-1,6,8,14a-tetrahydroxy-6a-methoxy-3-methyl-7,9,12,14-tetraoxo-5,6,6a,7,9,12,14,14a-octahydrobenzo[a]tetracene-2-carboxylate

### PHYSICO-CHEMICAL CHARACTERISTICS OF FACTOR F797 OF ANTIBIOTIC FIIRV 104/18 COMPLEX

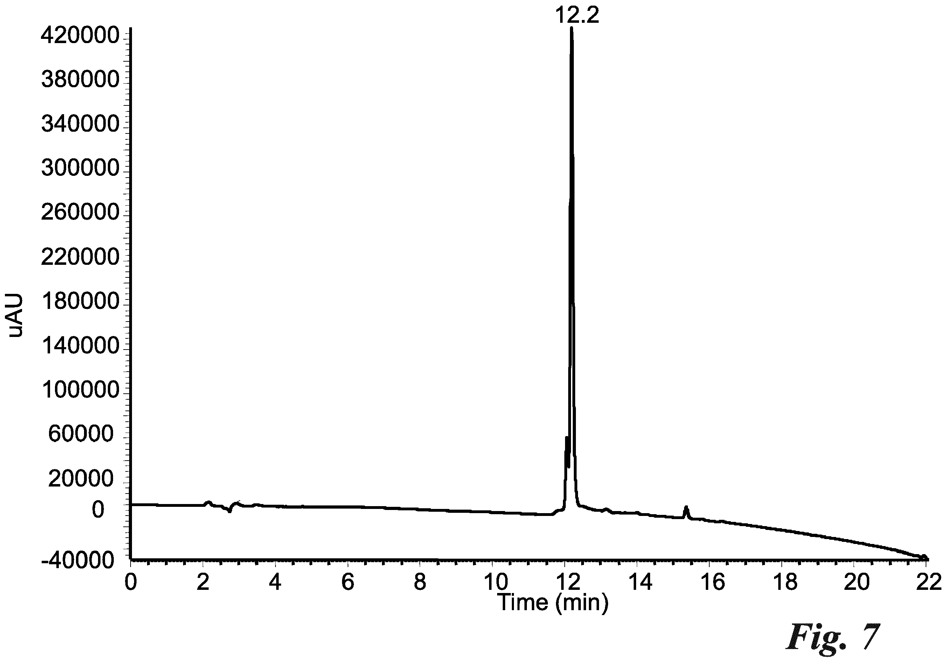

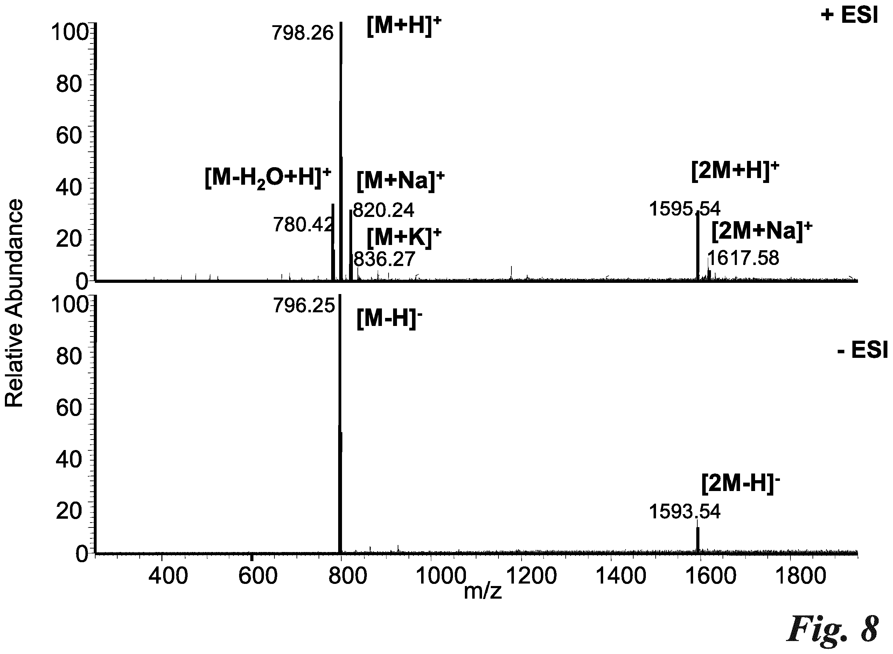

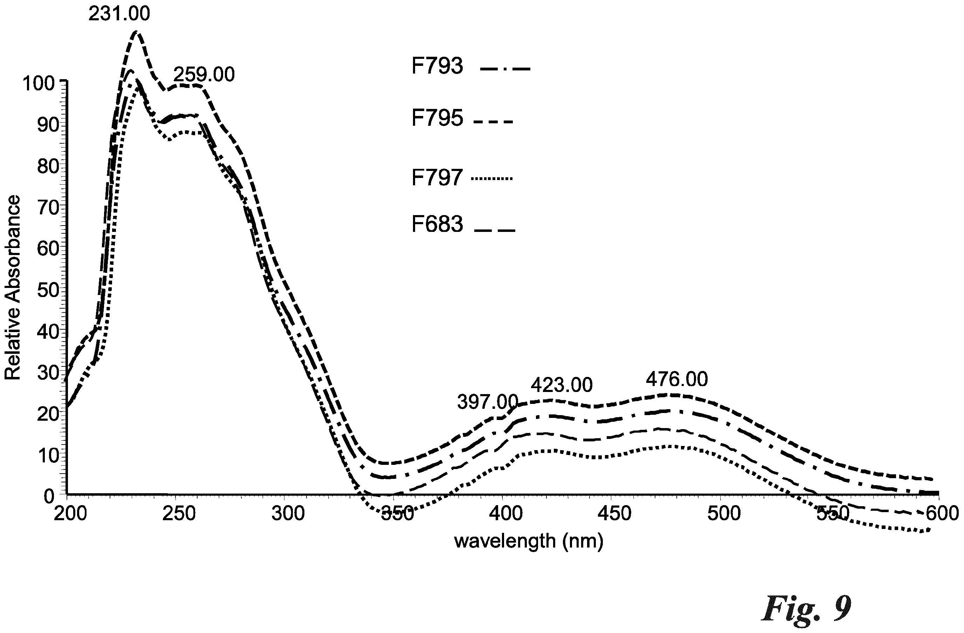

Factor F797 has a molecular weight of 797. The HPLC-MS analysis is shown in Figure 7.
*A)* Mass spectrometry: in MS experiments on a Thermo Scientific LTQ-xl instrument fitted with an electrospray source, using Thermo Scientific calibration mix, shows a mono-charged ion at m/z 798.26 corresponding to [M+H]⁺, a mono-charged ion at m/z 820.24 corresponding to [M+Na]⁺, a mono-charged ion at m/z 836.27 corresponding to [M+K]⁺, a mono-charged ion at m/z 780.42 corresponding to [M-H₂O+H]⁺, and a double-charged ion at m/z 1595.54 corresponding to [2M+H]⁺ a double-charged ion at m/z 1617.28 corresponding to [2M+Na]⁺. In negative mode factor F797 gives a mono charged ion at m/z 796.25 corresponding to [M-H]⁻ and a double-charged ion at m/z 1593.54 corresponding to [2M-H]⁻. In positive mode, the electrospray conditions are the following: spray voltage 2.7 kV; capillary temperature 275°C; capillary voltage 4.5 V; spectra can be obtained during a HPLC analysis or in infusion mode 10 µl/min. Spectra are recorded from a 0.1 mg/mL solution in CH₃CN/H₂O ratio 60/40 (v/v) and are reported in Figure 8 (full-scan low resolution spectrum).
B) MS/MS analysis of the mono charged ion is performed with the observed main fragmentations: mono charged ions at m/z 780, 766, 748, 716, 698, 666, 650, 634, 602, 524, 506, 474, 442 and 414.
C) The molecular weight of factor F797 is obtained from the analysis of high-resolution electrospray ionization acquired on an ESI-FT ICR SolariX instrument (Bruker Daltonik, Germany, GmbH). When the electrospray interface is set in negative ionization mode, the conditions are: spray voltage +3200 V; cap exit -210.0 V. Nitrogen is used as a drying at 3.7 liters/min and nebulizing gas at 1.0 bar; the ion transfer capillary is kept at 180°C. When the electrospray interface is in positive ionization mode, the conditions are: spray voltage -4300 V; cap exit +210.0; drying gas at 4 liters/min; nebulizing gas at 1.3 bar; the ion transfer capillary is kept at 200°C. Mass signals are recorded in the m/z 150-3000 mass range. Accurate mass calibration is performed in the range m/z 250-1000 by infusing a solution of sodium trifluoroacetate at the appropriate concentration. Sample solutions are introduced in the electrospray source using a syringe pump operating at 4 µl/min. Ions are observed at m/z 798.25962 [M+H]+ and 796.24458 [M-H]- (Table 1), error at <1.6 ppm. The deduced molecular formula is C₃₉H₄₃N₁O₁₇. The isotopic profile is coherent with the deduced molecular formula. CID fragmentation experiments are performed by evaluating different collision energy settings (from 15 to 30 V). Mass spectra are elaborated using the DataAnalysis software (Bruker Daltonics GmbH). When submitted to higher energy ions at m/z 524 are observed. In Table 1 measured values and the accuracy (ppm error) are reported.
D) The UV-Vis spectrum of factor F797, performed in 10 mM ammonium formate pH 4.5: CH₃CN (in ratio 50:50) with a Thermo Scientific Accela PDA detector, during a HPLC analysis, shows absorption maxima at 216, 228, 251, 300, 415 and 475nm, which indicates the presence of a complex naphthoquinone moiety. The spectrum is reported in Figure 9 where it is compared with the UV-Vis spectrum of the other factors. All antibiotic FIIRV 104/18 complex factors show the same UV-Vis spectrum.
E) ¹H-, ¹³C- and 2D NMR experiments are recorded in CD₃OD at 300°K on a Bruker Avance 500 MHz instrument. NMR spectra show signals consistent with the molecular formula C₃₉H₄₃N₁O₁₇. Compared to factor F795, carbon peak around 210 ppm in position 4" disappears as expected, and a new tertiary CH at 66.5 ppm is detected, typical for the new hydroxyl functional group.
   ¹H-NMR (500 MHz, CD₃OD): δ 8.10 (s, 1H), 6.60 (s, 1H), 5.94 (s, 1H), 5.33 (bs, 1H), 4.95 (bs, 2H), 4.04 (m, 1H), 3.80 (m, 1H), 3.79 (s, 3H), 3.77 (s, 3H), 3.70 (m, 1H), 3.68 (m, 1H), 3.57 (m, 1H), 3.53 (m, 3H), 3.38 (s, 3H), 3.27 (m, 1H), 2.35 (s, 3H), 2.03 (m, 2H), 1.68 (m, 2H), 1.32 (d, J = 6.0 Hz, 3H), 1.16 (d, J = 7.0 Hz, 3H).
   ¹³C-NMR (500 MHz, CD₃OD): δ 194.9, 190.7, 188.7, 179.3, 171.7, 161.7, 159.2, 147.7, 142.9, 142.3, 140.7, 134.3, 123.6, 120.7, 118.4, 115.3, 110.1, 103.1, 98.4, 84.9, 81.0, 79.1, 78.9, 77.9, 75.4, 72.5, 66.9, 66.5, 62.3, 61.4, 51.6, 51.4, 37.6, 25.2, 23.2, 22.3, 17.4, 15.9.
F) Determination of glycosidic moiety. Factor F797 is hydrolyzed under acidic conditions (1 or 6N HCl at 100°C for 1-24 hours) and the hydrolyzed mixture is analyzed by LC-MS. In these conditions the aglycone moiety C₂₆H₂₀O₁₂ without the amine with molecular weight 524 is generated, whose accurate mass is reported in Table 1. In MS/MS experiment at high resolution, a loss corresponding to 114.06808 is observed suggesting that the second sugar moiety present is L-rhodinose. Further neutral loss of 160.7298 corresponds to a C₇H₁₂O₄ moiety. Factor F797, factor F795 and the other antibiotic FIIRV 104/18 complex factors generate in MS/MS experiment the aglycone moiety with a Mw of 523 and molecular formula C₂₆H₂₁N₁O₁₁.

The data reported are consistent with the molecular formula and the chemical name reported below: 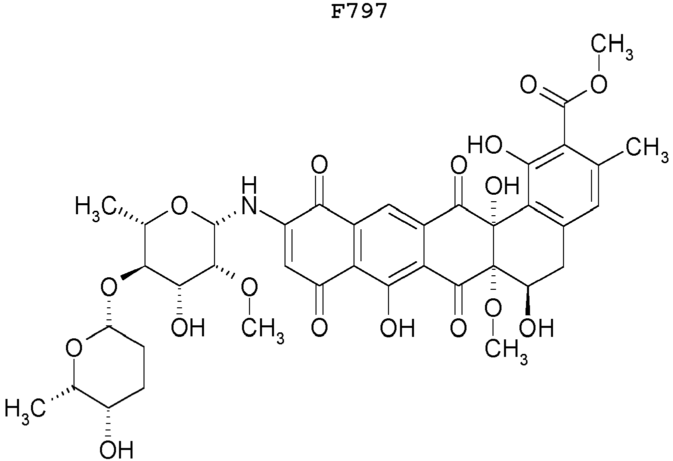 (6R,6aS,14aR)-methyl 11-[(2S,3R,4R,5R,6S)-4-hydroxy-5-{[(2R,5S,6S)-5-hydroxy-6-methyltetrahydro-2H-pyran-2-yl]oxy}-3-methoxy-6-methyltetrahydro-2H-pyran-2-ylamino]- 1,6,8,14a-tetrahydroxy-6a-methoxy-3-methyl-7,9,12,14-tetraoxo-5,6,6a,7,9,12,14,14a-octahydrobenzo[a]tetracene-2-carboxylate

### PHYSICO-CHEMICAL CHARACTERISTICS OF FACTOR F683

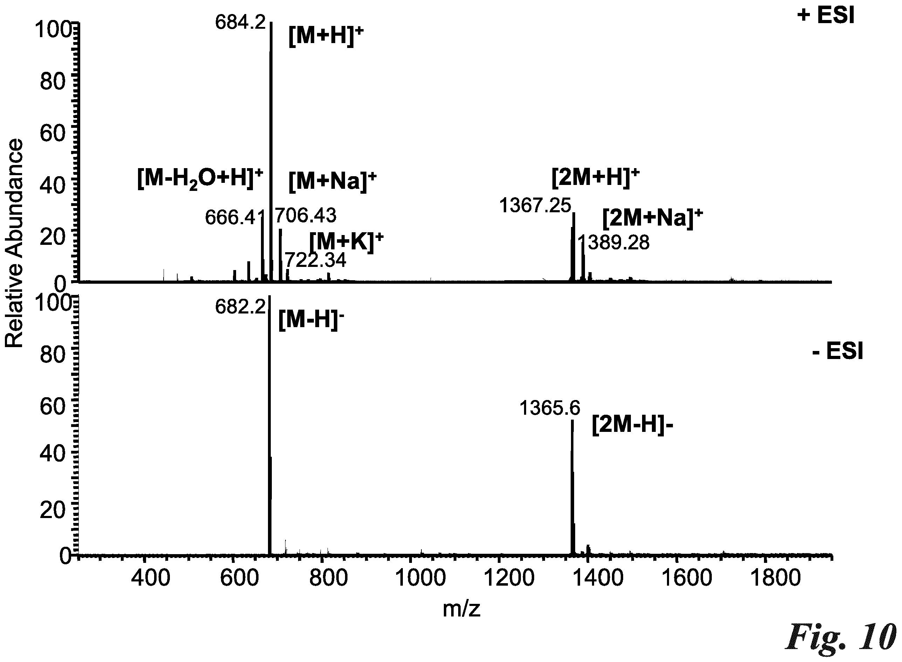

Factor F683 has a molecular weight of 683.
A) Mass spectrometry: in MS experiments on a Thermo Scientific LTQ- xl (Thermo Fisher Scientific Inc. CA, USA) instrument fitted with an electrospray source, using Thermo Scientific calibration mix, factor F683 shows a mono-charged ion at m/z 684.2 corresponding to [M+H]⁺, a mono-charged ion at m/z 706.43 corresponding to [M+Na]⁺, a mono-charged ion at m/z 722.34 corresponding to [M+K]⁺, mono-charged ion at m/z 666.41 corresponding to [M-H₂O+H]⁺ and a double-charged ion at m/z 1367.25 corresponding to [2M+H]⁺, a double-charged ion at m/z 1389.28 matching to [2M+Na]⁺. In negative mode factor F683 gives a mono charged ion at m/z 682.2 corresponding to [M-H]⁻ and a double-charged ion at m/z 1365.6 corresponding to [2M-H]⁻. In positive mode, the electrospray conditions are the following: spray voltage 2.7 kV; capillary temperature 275°C; capillary voltage 4.5 V; spectra can be obtained during a HPLC analysis or in infusion mode at 10 µl/min. Spectra are recorded from a 0.1 mg/mL solution in CH₃CN /H₂O 60/40(v/v) and are reported in Figure 10 (full-scan low resolution spectrum).
B) The molecular weight of factor F683 is obtained from the analysis of high-resolution electrospray ionization acquired on a ESI-FT ICR SolariX (Bruker Daltonics,Germany GmbH) which shows ions at *m*/*z* 684.19145 [M+H]+ and 682.17721 [M-H]⁻ error <1.5 ppm (Table 1). These data established the molecular formula as C₃₃H₃₃N₁O₁₅.
C) MS/MS analysis of the mono charged ion is performed with the observed main fragmentations: mono charged ions at m/z 666, 650, 634, 602, 524,506, 474, 442 and 414.
D) ¹H-, ¹³C- and 2D NMR experiments are recorded in CD₃OD at 300°K on a Bruker Avance 500 MHz NMR instrument. The spectra show signals consistent with the molecular formula C₃₃H₃₃N₁O₁₅ ¹H-NMR (500 MHz, CD₃OD): δ 8.12 (s, 1H), 6.64 (s, 1H), 5.99 (s, 1H), 4.96 (bs, 2H), 3.85 (s, 3H), 3.77 (s, 3H), 3.70 (m, 1H), 3.68 (m, 1H), 3.55 (m, 1H), 3.41 (m, 1H), 3.37 (bs, 3H), 3.36 (m, 1H), 3.30 (m, 1H), 2.42 (s, 3H), 1.29 (d, J = 6.0 Hz, 3H).
   ¹³C-NMR (500 MHz, CD₃OD): δ 194.9, 190.7, 188.8, 179.4, 171.7, 161.7, 159.2, 147.8, 142.9, 142.2, 140.6, 134.4, 123.5, 123.1, 120.7, 118.4, 115.2, 109.8, 104.2, 84.9, 80.5, 78.9, 78.8, 74.8, 73.7, 72.2, 62.3, 61.4, 51.6, 51.4, 37.5, 22.3, 16.6.
E) Determination of glycosidic moiety. Factor F683 is hydrolyzed under acidic conditions (1 or 6N HCl at 100°C for 1-24 hours) and the hydrolyzed mixture is analyzed by LC-MS. In these conditions the aglycone moiety C₂₆H₂₀O₁₂ without the amine with molecular weight 524 is generated, whose accurate mass is reported in Table 1. In MS/MS experiment the aglycone moiety found has a molecular weight of 523 and molecular formula C₂₆H₂₁N₁O₁₁.

The data reported are consistent with the molecular formula and the chemical name reported below: 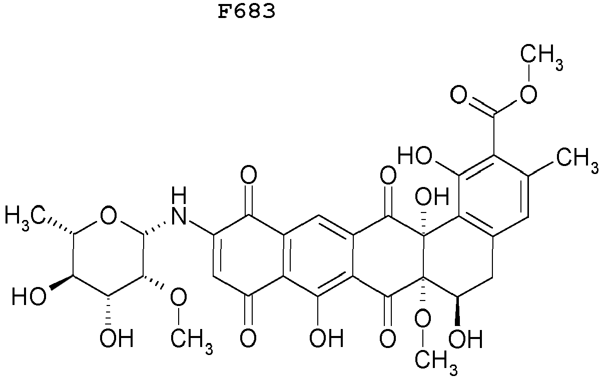 6R,6aS,14aR-methyl-11,6,8,14a-tetrahydroxy-11[(2S,3R, 4R,5R,6S)-4,5-dihydroxy-3-methoxy-6-methyltetrahydro-2H-pyran-2-ylamino]-6a-methoxy-3-methyl-7,9,12,14-tetraoxo-5,6,6a,7,9,12,14,14a-octahydrobenzo[a]tetracene-2-carboxylate

### PHYSICO-CHEMICAL CHARACTERISTICS OF FACTOR F793 OF ANTIBIOTIC FIIRV 104/18 COMPLEX

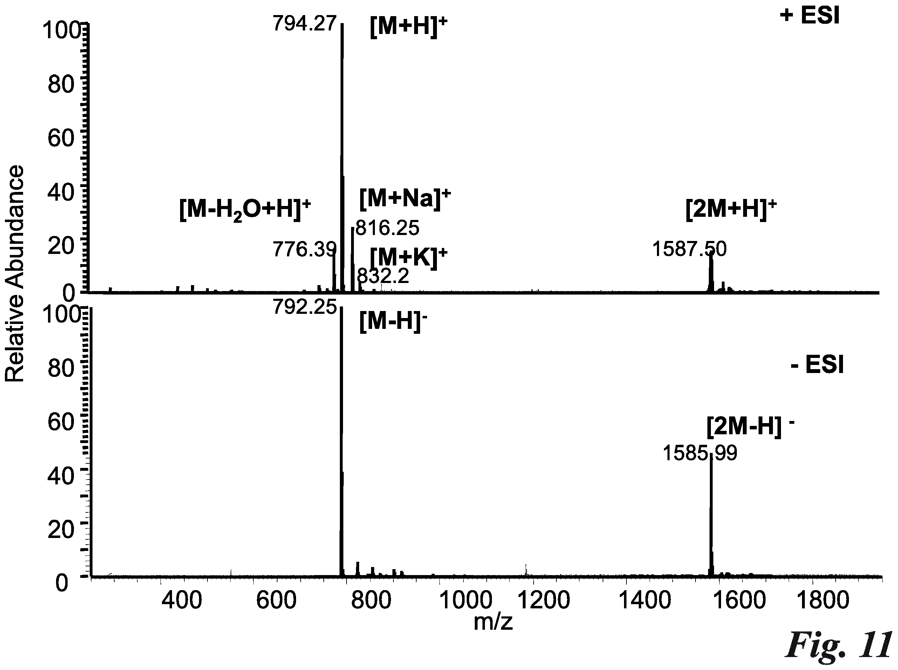

Factor F793 has a molecular weight of 793.
A) Mass spectrometry: in MS experiments on a Thermo Scientific LTQ-xl instrument (Thermo Fisher, Scientific Inc CA,USA) fitted with an electrospray source, using Thermo Scientific calibration mix, factor F793 shows a mono-charged ion at m/z 794.27 corresponding to [M+H]⁺, a mono-charged ion at m/z 816.25 corresponding to [M+Na]⁺, a mono-charged ion at m/z 832.27 corresponding to [M+K]⁺, mono-charged ion at m/z 776.39 corresponding to [M-H₂O+H]⁺ and a double-charged ion at m/z 1587.50 matching to [2M+H]⁺. In negative mode factor F793 gives a mono-charged ion at m/z 792.25 corresponding to [M-H]⁻ and a double-charged ion at m/z 1585.99 corresponding to [2M-H]⁻ In positive mode, the electrospray conditions are the following: spray voltage 2.7 kV; capillary temperature 275°C; capillary voltage 4.5 V; spectra can be obtained during a HPLC analysis or in infusion mode at 10 µl/min. Spectra are recorded from a 0.1 mg/mL solution in CH₃CN/H₂O 60/40(v/v) and are reported in Figure 11 (full-scan low resolution spectrum). The suggested molecular formula is C₃₉H₃₉N₁O₁₇
B) MS/MS analysis of the mono charged ion is performed with the observed main fragmentations: mono charged ions at m/z 776, 762, 746, 732, 714, 696, 666, 650, 634, 602, 524, 506, 474, 442 and 414. In MS/MS experiment at high resolution a loss corresponding to 110.03678 is observed suggesting that the sugar moiety is L-aculose.
C) ¹H-, ¹³C- and 2D NMR experiments are recorded in CDCl₃ and CD₃OD at 300°K on a Bruker Avance 500 MHz NMR instrument. NMR spectrum shows signals, consistent with the molecular formula C₃₉H₃₉N₁O₁₇.
D) ¹H-NMR (500 MHz, CDCl₃): δ 14,14 (s, 1H), 12.09 (s, 1H), 8.25 (s, 1H), 6.89 (dd, *J* = 10.5, 3.0 Hz, 1H), 6.80 (d, *J* = 9.0 Hz, 1H), 6.56 (s, 1H), 6.13 (d, *J* = 10.5 Hz, 1H), 5.92 (s, 1H), 5.76 (d, *J* = 3.0 Hz, 1H), 4.99 (d, *J* = 6.5 Hz, 1H), 4.74 (d, *J* = 9.0 Hz, 1H), 4.59 (q, *J* = 7.0 Hz, 1H), 3.87 (s, 3H), 3.85 (m, 1H), 3.82 (s, 3H), 3.72 (m, 2H), 3.59 (m, 1H), 3.43 (s, 3H), 3.41 (m, 1H), 3.36 (m, 1H), 2.42 (s, 3H), 1.41 (d, *J* = 7.0 Hz, 3H), 1.39 (d, *J* = 7.0 Hz, 3H).
   ¹H-NMR (500 MHz, CD₃OD): δ 8.12 (s, 1H), 7.02 (dd, *J* = 11.0, 3.5 Hz, 1H), 6.63 (s, 1H), 6.06 (d, *J=* 11.0 Hz, 1H), 5.98 (s, 1H), 5.74 (d, *J =* 3.5 Hz, 1H), 4.97 (bs, 2H), 4.62 (q, *J =* 7.0 Hz, 1H), 3.86 (m, 1H), 3.84 (s, 3H), 3.80 (s, 3H), 3.77 (m, 1H), 3.70 (m, 1H), 3.52 (m, 2H), 3.38 (s, 3H), 3.28 (m, 1H), 2.42 (s, 3H), 1.32 (m, 6H).
   ¹³C-NMR (500 MHz, CD₃OD): δ 211.5, 197.2, 190.7, 188.7, 179.4, 171.7, 161.6, 159.2, 147.7, 144.2, 142.9, 142.2, 140.6, 134.3, 125.9, 123.6, 123.1, 120.7, 118.4, 115.2, 110.1, 103.2, 94.5, 84.9, 81.2, 79.3, 79.0, 78.9, 75.1, 72.1, 70.1, 62.3, 61.3, 51.4 (2C), 37.5, 22.4, 17.1, 13.9.

The data reported are consistent with the molecular formula and the chemical name reported below: 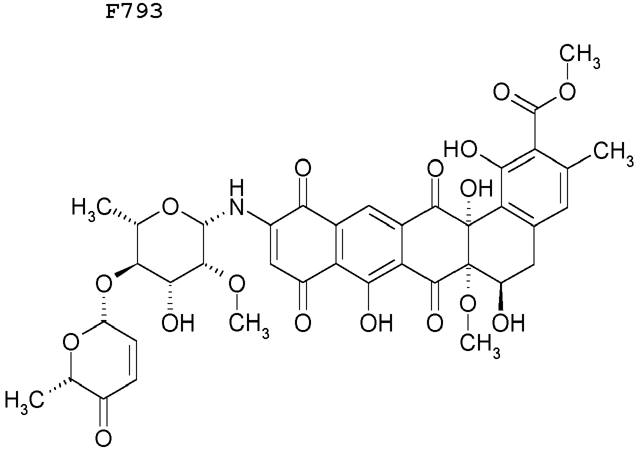 (6R,6aS,14aR)-methyl 11-[(2S,3R,4R,5R,6S)-4-hydroxy-3-methoxy-6-methyl-5-{[(2S,6S)-6-methyl-5-oxo-5,6-dihydro-2H-pyran-2-yl]oxy}tetrahydro-2H-pyran-2-ylamino]-1,6,8,14a-tetrahydroxy-6a-methoxy-3-methyl-7,9,12,14-tetraoxo-5,6,6a,7,9,12,14,14a-octahydrobenzo[a]tetracene-2-carboxylate

### PHYSICO-CHEMICAL CHARACTERISTICS OF FACTOR F813 OF ANTIBIOTIC FIIRV 104/18 COMPLEX

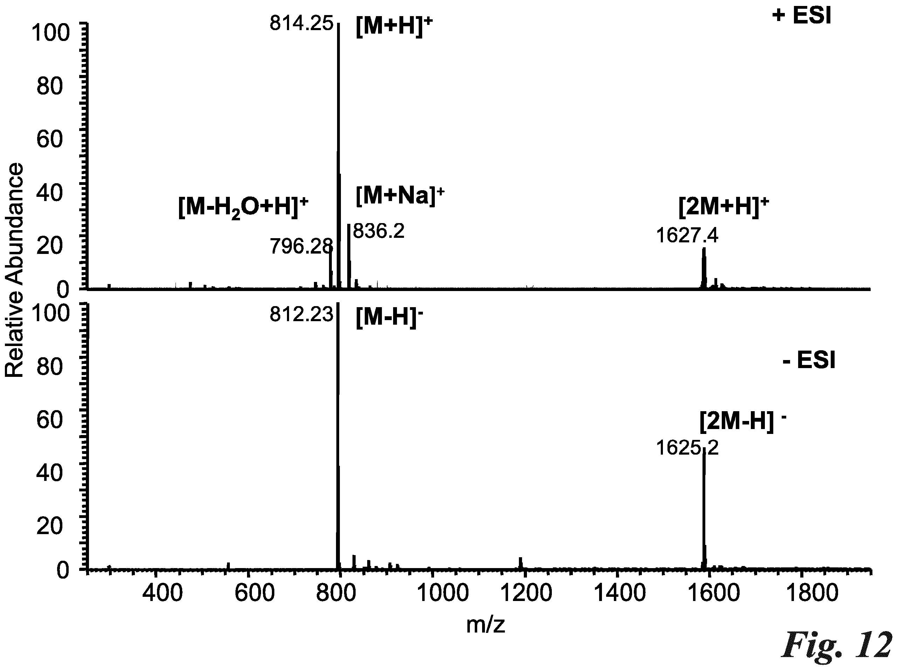

Factor F813 has a molecular weight of 813.
A) Mass spectrometry: in MS experiments on a Thermo Scientific LTQ-xl (Thermo Fisher Scientific Inc. USA) instrument fitted with an electrospray source, using Thermo Scientific calibration mix, factor F813 gives a protonated mono-charged ion at m/z 814.24 corresponding to [M+H]⁺, a mono-charged ion at m/z 836.2 corresponding to [M+Na]⁺, mono-charged ion at m/z 796.28 corresponding to [M-H₂O+H]⁺ and a double-charged ion at m/z 1627.4 corresponding to [2M+H]⁺. In negative mode factor F813 gives a mono charged ion at m/z 812.23 corresponding to [M-H]⁻ and a double-charged ion at m/z 1625.2 corresponding to [2M-H]⁻. In positive mode, the electrospray conditions are the following: spray voltage 2.7 kV; capillary temperature 275°C; capillary voltage 4.5 V; spectra can be obtained during a HPLC analysis or in infusion mode 10 µl/min. Spectra are recorded from a 0.1 mg/mL solution in CH₃CN/water 60/40 (v/v) and are reported in Figure 12.
B) MS/MS analysis of the mono charged ion is performed with the observed main fragmentations: mono charged ions at m/z 796, 782, 764, 746, 732, 714, 696, 666, 650, 634, 602, 524, 506, 474, 442 and 414.
C) The molecular weight of factor F813 is obtained from the analysis of high-resolution electrospray ionization acquired on a ESI-FT ICR SolariX Instrument (Bruker Daltonics, Germany, GmbH) as described above which shows ions at *m*/*z* 814.25452 [M+H]+ and 812.23963 [M-H]⁻ error < 1.2 ppm (Table 1). These data establish the molecular formula as C₃₉H₄₃N₁O₁₈.
D) ¹H-, ¹³C- and 2D NMR experiments are recorded in CD₃OD at 300°K on a Bruker Avance 500 MHz NMR instrument. NMR spectrum shows signals, consistent with the molecular formula C₃₉H₄₃N₁O₁₈.
   ¹H-NMR (500 MHz, CD₃OD): δ 8.12 (s, 1H), 6.64 (s, 1H), 5.99 (s, 1H), 5.43 (t, *J =* 2.5 Hz, 1H), 4.96 (bs, 2H), 4.00 (m, 1H), 3.92 (m, 1H), 3.85 (s, 3H), 3.82 (m, 1H), 3.77 (s, 3H), 3.65 (m, 1H), 3.57 (m, 1H), 3.53 (m, 3H), 3.38 (s, 3H), 3.28 (m, 1H), 2.42 (s, 3H), 1.93 (m, 2H), 1.32 (d, J = 6.0 Hz, 3H), 1.23 (d, *J* = 6.5 Hz, 3H).
   ¹³C-NMR (500 MHz, CD₃OD): δ 195.0, 190.7, 188.7, 179.4, 171.7, 161.7, 159.2, 147.8, 142.9, 142.2, 140.6, 134.4, 123.6, 123.5, 120.7, 118.4, 115.2, 110.2, 103.1, 99.2, 84.9, 81.1, 79.1, 78.9, 78.2, 75.4, 72.4, 70.9, 66.8, 65.5, 62.3, 61.4, 51.6, 51.4, 37.6, 31.9, 22.3, 17.4, 15.7.

The data reported are consistent with the molecular formula and the chemical name reported below: 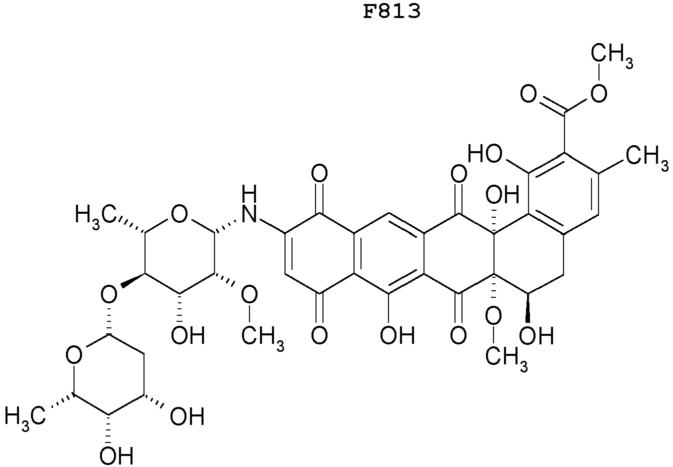 (6R,6aS,14aR)-methyl 11-[(2S,3R,4R,5R,6S)-5-{ [(2R,5S,6S)-4,5-dihydroxy-6-methyltetrahydro-2H-pyran-2-yl]oxy}-4-hydroxy-3-methoxy-6-methyltetrahydro-2H-pyran-2-ylamino]-1,6,8,14a-tetrahydroxy-6a-methoxy-3-methyl-7,9,12,14-tetraoxo-5,6,6a,7,9,12,14,14a-octahydrobenzo[a]tetracene-2-carboxylate.

### IN VITRO BIOLOGICAL ACTIVITY OF ANTIBIOTIC FIIRV 104/18 COMPLEX AND ITS INDIVIDUAL FACTORS

Antimicrobial activity of the antibiotic FIIRV 104/18 complex is determined by the broth microdilution method according to the procedures published by the Clinical and Laboratory Standards Institute (CLSI), formerly the National Committee for Clinical Laboratory Standards (NCCLS) (document M07-A9). Inocula are: 2-5×10⁵ CFU/mL for Gram positive and Gram negative bacteria; 10⁴ CFU/mL for *Candida albicans.*

The media used are: cation adjusted Mueller Hinton broth (CAMHB) for *Staphylococci, Enterococci, Moraxella catarrhalis, Escherichia coli;* Todd Hewitt broth (THB) for *Streptococci;* RPMI 1640 Medium for *C*. *albicans.* The effect of 30% bovine serum is determined under the same experimental conditions. Cultures are incubated at 35°C in air. After 18-24 hours visual readings are performed and MICs determined. The MIC (Minimal Inhibitory Concentration) is defined as the lower concentration of antibiotic at which there is no visible growth. The strains used are clinical isolates or strains from American Type Culture Collection (ATCC). Individual factors of antibiotic FIIRV 104/18 are dissolved in DMSO to obtain a 1000 ug/mL stock solution, and subsequently diluted in water to obtain working solution. The results of the tests are reported in Table 3.

Antibiotic FIIRV 104/18 factors F793, F795, F797 and F813 show very high antibacterial activity against Gram positive bacteria such as: *Staphylococci* including methicillin resistant *S. aureus* (MRSA) and multi-resistant (MR) coagulase-negative *Staphylococci, Streptococci* and *Enterococci,* including Vancomycin Resistant (VanA) strains. The Gram negative *Moraxella catarrhalis* is susceptible to antibiotic FIIRV 104/18 individual factors. Interestingly, FIIRV 104/18 individual factors resulted active in the range between 0.25 and 8 ug/mL also on sensitive and resistant clinical isolates of *Enteroccocus faecalis* while the antibiotic SF2446 showed MIC values of 50-100 pg/mL.

In addition to what is reported under section "Background of the invention", it is emphasized that the European Antimicrobial Resistance Surveillance System (EARSS) data suggest that the burden of bacterial bloodstream infection has been increasing in Europe for *Staphylococcus aureus, Escherichia coli, Streptococcus pneumoniae, Enterococcus faecalis* and *Enterococcus faecium.* Apparently, infections with resistant clones add to rather than replace infections caused by susceptible bacteria [de Kraker M.E., Jarlier V, Monen J.C., et al., "The changing epidemiology of bacteraemias in Europe: trends from the European Antimicrobial Resistance surveillance System", Clin. Microbiol. Infect,19(9):860-8 (2013)]. *Staphylococcus aureus* can cause life-threatening infections and MRSA are associated with considerable morbidity and mortality, prolonging the duration of stay and increasing hospitalization costs. The majority of MRSA strains are resistant to several of the most commonly used antimicrobial agents, including β-lactams antibiotics, macrolides, aminoglycosides, the latest generation of cephalosporins and glycopeptides [Appelbaum, P. C., "Reduced glycopeptide susceptibility in methicillin resistant Staphylococcus aureus (MRSA)", Int. J. Antimicrob. Agents 30:398-408 (2007)]. The CDC reported that MRSA is one of the most common causes of healthcare-associated infections and the MRSA infections are increasing in community settings. In 2011, 80,461 cases were reported and 11,285 reported deaths related to MRSA [CDC 2013a report "Antibiotic Resistance Threats in the United States" 2013]. *Enterococcus faecium* has been highlighted by the Infectious Diseases Society of America as one of the key problem bacteria, or ESKAPE (*Enterococcus faecium, Staphylococcus aureus, Klebsiella pneumoniae, Acinetobacter baumannii, Pseudomonas aeruginosa,* and *Enterobacter* species) pathogens, requiring new therapies [Boucher H.W., Talbot G.H., Bradley J.S., Edwards J.E., et.al,. "Bad bugs, no drugs: no ESKAPE! An update", Clin. Infect. Dis. 48:1-12 (2009)] and there has been a steadily increasing prevalence of *E. faecium* related nosocomial infections [Hidron A.I., Edwards J.R., Patel J., et al., "NHSN annual update: antimicrobial-resistant pathogens associated with healthcare associated infections: annual summary of data reported to the National Healthcare Safety Network at the Centers for Disease Control and Prevention, 2006-2007", Infect. Control Hosp. Epidemiol. 29:996-1011(2008)]. Large amounts of health care funding are spent trying to control antibiotic-resistant bacteria in hospitals globally, yet in many institutions around the world, vancomycin-resistant E. faecium (VREfm) infections continue to rise [Johnson P.D., Ballard S.A., Grabsch E.A., Stinear T.P., et al., "A sustained hospital outbreak of vancomycin-resistant Enterococcus faecium bacteremia due to emergence of vanB E. faecium sequence type 203", J. Infect. Dis. 202:1278-1286 (2010)]. The rapid increase of vancomycin resistance compromises physicians' ability to treat infections caused by many of these strains because often no other antimicrobial drugs are available.

*Streptococcus pneumoniae* is the most important pathogen in otitis, sinusitis, bronchitis, and community-acquired pneumonia, as well as a predominant cause of meningitis and bacteraemia. Over the past three decades, antimicrobial resistance in *Streptococcus pneumoniae* has dramatically increased worldwide. Most of these strains show resistance to multiple antibiotics and the dissemination of antibiotic-resistant pneumococci is observed in Southern and Eastern Europe, North America, South America, Africa, and Asia. *Moraxella catarrhalis* is an important and common human respiratory tract pathogen, in particular as a cause of acute otitis media in children and of exacerbations in adults with chronic obstructive pulmonary disease [Murphy T.F., Parameswaran G.I., "Moraxella catarrhalis, a Human Respiratory Tract Pathogen", Clinical Infectious Diseases 49:124-31(2009)].

### IN VIVO BIOLOGICAL ACTIVITY OF ANTIBIOTIC FIIRV 104/18 FACTOR F795

Female ICR mice (Harlan, Italy) weighing 21-24 g are infected intraperitoneally (ip) with 5 × 10⁴ cells of *Staphylococcus aureus* Smith ATCC 19636 in 0.5 mL of 5% gastric mucine. Antibiotic FIIRV 104/18 factor F795 is administered intravenously (iv) within 10-15 min after infection. The 50% effective dose (ED50) is calculated by the Spearman-Kärber method [Finney D.J., "The Spearman-Kärber method". In: Statistical Methods in Biological Assay, 524-530, (1952) Charles Griffin &Co., Ltd., London]. Antibiotic FIIRV 104 factor F795 has an iv ED₅₀ value of 0.80 mg/kg (95% confidence limits 0.61-1.05). Vancomycin, used as reference drug, in the same experiment, has an iv ED₅₀ value of 0.93 mg/kg (95% confidence limits 0.77-1.13).

### TABLES

**Table 1. Mass values measured by high-resolution electrospray ionization of the antibiotic FIIRV 104/18 factors, of the hydrolysis products and of glycosidic molecular ion obtained at higher energy. The assigned molecular formula and the accuracy are reported; m is the molecular weight, exp. m/z is the measured value of the ions at different charge status, z is the charge of the ions. Theor m/z is the theoretical value and error in ppm is the accuracy of the measurement.**

| **m** | **exp. m/z** | **z** | **Ion molecular formula** | **theor. m/z** | **error (ppm)** | **Compound molecular formula** |
|---|---|---|---|---|---|---|
| 795 | 796.24576 | 1 | C₃₉H₄₂N₁O₁₇ | 796.24473 | 1.3 | C₃₉H₄₁N₁O₁₇ |
| | 794.22955 | -1 | C₃₉H₄₀N₁O₁₇ | 794.23017 | 0.8 | |
| 797 | 798.25962 | 1 | C₃₉H₄₄N₁O₁₇ | 798.26038 | 0.9 | C₃₉H₄₃N₁O₁₇ |
| | 796.24458 | -1 | C₃₉H₄₂N₁O₁₇ | 796.24582 | 1.6 | |
| 813 | 814.25452 | 1 | C₃₉H₄₄N₁O₁₈ | 814.25529 | 1 | C₃₉H₄₃N₁O₁₈ |
| | 812.23963 | -1 | C₃₉H₄₂N₁O₁₈ | 812.23964 | 0.1 | |
| 683 | 684.19145 | 1 | C₃₃H₃₄N₁O₁₅ | 684.19230 | 1.2 | C₃₃H₃₃N₁O₁₅ |
| | 682.17721 | -1 | C₃₃H₃₂N₁O₁₅ | 682.17774 | 0.8 | |
| 523 | 524.11767 | 1 | C₂₆H₂₂N₁O₁₁ | 524.11874 | 2 | C₂₆H₂₁N₁O₁₁ |
| 524 | 525.10221 | 1 | C₂₆H₂₁O₁₂ | 525.10275 | 1 | C₂₆H₂₀O₁₂ |
| | 523.08774 | -1 | C₂₆H₁₉₁O₁₂ | 523.08710 | 1 | |

**Table 2. Factor F795 NMR data. ¹H-, ¹³C- and 2D NMR experiments are recorded in CDCl₃ at 300°K on a Bruker Avance 500 MHz.**

| **Positio n** | **¹H** | **IN T** | **MULT (J)** | **¹³C** | **HMBC** | **COSY** | **NOESY** |
|---|---|---|---|---|---|---|---|
| | δ | | Hz | δ | | | |
| 1 | | | | 160.08 | | | |
| 2 | | | | 109.86 | | | |
| 3 | | | | 142.88 | | | |
| 4 | 6.56 | 1 | s | 124.32 | C-1, - 2, -3, -4a, - 5, - 14a, - 14b, - 16 | H-5, H-16 | H-5, H-16 |
| 4a | | | | 143.27 | | | |
| 5-A | 3.59 | 1 | m | 38.10 | C-4a, - 6, -6-OCH₃, - 14b | H-4, H-6 | H-4, H-6 |
| 5-B | 3.36 | 1 | m | 38.10 | C-4a, - 6, -6-OCH₃, - 14b | H-4, H-6 | H-4, H-6 |
| 6 | 4.98 | 1 | d (7.0) | 62.99 | C-4a, - 5, -6a, -7, - 14a | H-5 | H-5A, H-5B, H-6a(OCH₃) |
| 6a | | | | 84.62 | | | |
| 6a-OCH3 | 3.42 | 3 | s | 52.66 | C-6a | | H-6 |
| 7 | | | | 190.07 | | | |
| 7a | | | | 123.99 | | | |
| 8 | | | | 162.39 | | | |
| 8a | | | | 118.62 | | | |
| 9 | | | | 188.70 | | | |
| 10 | 5.90 | 1 | s | 104.25 | C-8, - 8a, -9, -11, - 12 | | H-1' |
| 11 | | | | 147.09 | | | |
| 12 | | | | 179.32 | | | |
| 12a | | | | 133.90 | | | |
| 13 | 8.27 | 1 | s | 116.18 | C-7a, - 8, -8a, -9, - 12, -14 | | |
| 13a | | | | 140.16 | | | |
| 14 | | | | 196.24 | | | |
| 14a | | | | 78.84 | | | |
| 14b | | | | 119.78 | | | |
| 15 | | | | 172.11 | | | |
| 15-OCH3 | 3.86 | 3 | s | 52.37 | C-15 | | |
| 16 | 2.42 | 3 | s | 23.92 | C-2, - 3, -4 | H-4 | H-4 |
| 1' | 4.74 | 1 | d (9.0 ) | 79.51 | C-2' | NH-11 | H-10, NH-11, H-2', H-5' |
| 2' | 3.70 | 1 | m | 80.60 | C-1', - 3' | H-3', H-4' | H-2' (OCH₃) |
| 2'-OCH3 | 3.81 | 3 | s | 62.75 | C-2' | | H-6', H-1" |
| 3' | 3.83 | 1 | m | 75.62 | C-2', - 4' | H-2', H-4' | H-2', H-5' |
| 4' | 3.70 | 1 | m | 77.60 | C-3', - 5', 6', -1" | H-3', H-5' | H-1", H-6' |
| 5' | 3.41 | 1 | m | 72.75 | C-4' | H-4', H-6' | H-1', H-6' |
| 6' | 1.36 | 3 | d (7.0 ) | 17.85 | C-4', - 5' | H-5' | H-5', H-4' |
| 1" | 5.57 | 1 | dd | 97.86 | C-5" | H-2" | H-4', H-2", H-3", H-2'(OCH₃) |
| 2" | 1.98/ 2.44 | 1 | m | 27.69 | C-1", -3", 4" | H-1", H-3" | H-1", H-3" |
| 3" | 2.46 | 2 | m | 33.34 | C-2", -4" | H-3" | H-2", H-3" |
| 4" | | | | 211.04 | | | |
| 5" | 4.31 | 1 | q (7.0 ) | 71.25 | C-1", -4", - 6" | H-6" | H-6" |
| 6' ' | 1.31 | 3 | d (7.0 ) | 14.69 | C-4", -5" | H-5" | H-5" |
| NH-11 | 6.82 | 1 | d (9.0 ) | | C-10 | H-1' | |
| OH-1 | 12.08 | 1 | s | | | | |
| OH-8 | 14.16 | 1 | s | | | | |

**Table 3. Antimicrobial activity reported as MIC (pg/mL) of antibiotic FIIRV 104/18 factors F793, F795, F797, F813 compared with F683 factor, vancomycin (V), ciprofloxacin (C), Amphotericin B (AmB). The symbol - means not determined; SB=bovine serum. Inocula are: 2-5×10⁵ CFU/mL for Gram positive and Gram negative bacteria; 10⁴ CFU/mL for Candida albicans; medium: CAMHB; THB S. pyogenes and S. pneumoniae; RPMI C. albicans.**

| **TABLE 3** | **MIC (µg/mL)** | | | | | | | |
|---|---|---|---|---|---|---|---|---|
| **Microorganism** | **F793** | **F795** | **F797** | **F813** | **F683** | **V** | **C** | **AmB** |
| L1400 S.aureus MRSA | 0.5 | 0.015 | 0.015 | 0.5 | 0.5 | 1 | 0.125 | - |
| L819 S. aureus Smith ATCC19636 | 0.5 | 0.002 | 0.002 | 0.125 | 0.5 | 1 | 0.06 | - |
| L819 S. aureus Smith, + 30 % SB | 4 | 0.125 | 0.125 | 1 | 8 | 1 | 0.06 | - |
| L100 S.aureus ATCC 6538 | 1 | 0.008 | 0.008 | 0.125 | 2 | 1 | 0.5 | - |
| L1577 S. epidermidis | 0.25 | 0.015 | 0.015 | 0.5 | 0.5 | 2 | 0.125 | - |
| L1730 S. haemolyticus | 1 | 0.015 | 0.015 | 0.5 | 0.5 | 2 | 0.25 | - |
| L1729 S. haemolyticus MDR | 1 | 0.015 | 0.015 | 0.5 | 0.5 | 2 | 8 | - |
| L49 S. pyogenes C203 | 0.03 | 0.015 | 0.002 | ≤ 0-125 | 0.125 | 0.5 | 0.25 | - |
| L44 S. pneumoniae | 0.06 | 0.015 | 0.004 | 0.125 | 0.125 | 0.5 | 1 | - |
| L568 E. faecium | 1 | 0.25 | 0.5 | 4 | 16 | 2 | 2 | - |
| L569 E. faecium VanA | 1 | 0.25 | 0.5 | 4 | 16 | > 128 | 2 | - |
| L569 E. faecium VanA, + 30% SB | 16 | 2 | 2 | 16 | > 32 | > 128 | 2 | |
| L559 E. faecalis | 0.5 | 0.25 | 0.25 | 4 | 8 | 1 | 0.5 | - |
| L560 E. faecalis VanA | 0.5 | 0.125 | 0.125 | 4 | 8 | > 128 | 0.5 | - |
| L560 E. faecalis VanA, + 30% SB | 2 | 0.5 | 0.5 | 16 | 32 | > 128 | 0.5 | |
| L3294 M. catarrhalis | 1 | 0.125 | 0.06 | 0.5 | 0.5 | > 2 | 0.016 | - |
| L47 E. coli, SKF 12140 | > 128 | > 128 | 64 | 128 | > 128 | > 2 | 0.002 | - |
| L2048 B. cenocepacia | 128 | 32 | 16 | 64 | > 128 | - | - | - |
| L3030 A. baumannii | 128 | 16 | 16 | 128 | > 128 | - | 0.5 | - |
| L145 C. albicans | > 128 | > 128 | > 128 | > 128 | > 128 | - | - | 1 |

### BRIEF DESCRIPTION OF THE FIGURES

Figure 1 shows the LC-MS (Liquid Chromatography-Mass Spectrometry) analysis of the factor F795.
Figure 2 shows the full-scan low resolution spectrum of factor F795. The adducts ions are identified and assigned.
Figure 3 represents the UV-visible spectrum of factor F795 dissolved in CH₃CN:NH₄COOH 1 : 1.
Figure 4 represents 'H-NMR spectrum of factor F795 recorded in CDCl₃ at 300°K on a Bruker Avance 500 MHz spectrometer.
Figure 5 represents the ¹³C-NMR spectrum of factor F795 recorded in CDCl₃ at 300°K on a Bruker Avance 500 MHz spectrometer.
Figure 6 represents the overall stereostructure of factor F795 structure.
Figure 7 shows the LC-MS (Liquid Chromatography-Mass Spectrometry) analysis of the Factor of F797.
Figure 8 shows the full-scan low resolution spectrum of factor F797. The adducts ions are identified and assigned.
Figure 9 represents the UV spectrum of factors F795, F797, F793, F683 dissolved in CH₃CN:NH₄COOH 1:1.
Figure 10 shows the full-scan low resolution spectrum of factor F683. The adducts ions are identified and assigned.
Figure 11 shows the full-scan low resolution spectrum of factor F793. The adducts ions are identified and assigned.
Figure 12 shows the full-scan low resolution spectrum of antibiotic F813. The adducts ions are identified and assigned.

### EXAMPLES

The Examples set forth below are for illustrative purposes only and are not intended to limit, in any way, the scope of the present invention.

### EXAMPLE 1

### ANALYTICAL METHODS AND MATERIALS

Unless otherwise noted, reagents and solvents are used as received from commercial suppliers. Proton nuclear magnetic resonance (NMR) spectra are obtained on Bruker Avance spectrometer at 500MHz. Spectra are given in ppm (δ) and coupling constants, J, are reported in Hertz. Tetramethylsilane (TMS) is used as an internal standard.

Mass spectra are collected using as LC-MS instrument a Thermo Scientific LTQ-xl instrument (Thermo Fisher, Scientific Inc. CA,USA) fitted with a ion trap electrospray ionization (ESI) source.

Accurate mass measurements are collected using a ESI-FT-ICR Solarix instrument (Bruker Daltonics, Germany, GmbH). HPLC analyses are obtained using a Luna C18(2) column (250×4.6 mm, Phenomenex, Torrance, Calif.) or a Symmetry C18 column 250×4.6 mm, (Waters; Milford MA, USA) with Thermo Scientific Accela PDA detector or UV detection at 223 nm using a standard solvent gradient program where Phase A (A) is 10mM ammonium formate pH 4.5, and Phase B (B) is CH₃CN. Here below are reported the solvent gradient program of the two methods used (Method 1 or Method 2).

### Method 1:

| Time (min) | Flow (mL/min) | %A | %B |
|---|---|---|---|
| 0 | 1.0 | 75 | 25 |
| 35 | 1.0 | 10 | 90 |
| 40 | 1.0 | 10 | 90 |
| 45 | 1.0 | 75 | 25 |

### Method 2:

| Time (min) | Flow (mL/min) | % A | % B |
|---|---|---|---|
| 0 | 1.0 | 60 | 40 |
| 3 | 1.0 | 60 | 40 |
| 15 | 1.0 | 45 | 55 |
| 17.5 | 1.0 | 10 | 90 |
| 20.5 | 1.0 | 10 | 90 |
| 21 | 1.0 | 60 | 40 |

The preparative HPLC are carried out by using a Shimadzu AP-20 liquid chromatograph (Shimadzu Corporation, Japan). UV data are acquired at 254 nm and 360 nm and the below reported solvent gradient program is used: (A) is 20 mM ammonium formate pH 4.5, and B is CH₃CN.

### Preparative Method:

| Time (min) | Flow (mL/min) | % A | %B |
|---|---|---|---|
| 0 | 8.0 | 65 | 35 |
| 2 | 20.0 | 65 | 35 |
| 8 | 20.0 | 65 | 35 |
| 14 | 20.0 | 58 | 42 |
| 19 | 20.0 | 58 | 42 |
| 36 | 20.0 | 45 | 55 |
| 40 | 20.0 | 30 | 70 |
| 42 | 20.0 | 10 | 90 |
| 45 | 20.0 | 10 | 90 |
| 48 | 20.0 | 65 | 35 |

### EXAMPLE 2: FERMENTATION METHOD OF DACTYLOSPORANGIUM FIIRV SP. 104/18 DSM 32068

*Dactylosporangium* FIIRV sp. 104/18 DSM 32068 strain is maintained on oatmeal agar (ISP medium 3) plates for 3-4 weeks at 28°C. The microbial content of one plate is scraped with 5 mL sterile water and inoculated into 500 mL Erlenmeyer flasks containing 100 mL of seed medium (AF/MS) which is composed of (g/L) : 20 g dextrose monohydrate, 2 g yeast extract, 8 g soybean meal, 1 g of NaCl and 4 g of CaCO₃. The medium is prepared in deionized-water and pH adjusted to 7.3 prior to sterilization at 121°C for 20 minutes. The inoculated flasks are grown at 28 °C, on a rotatory shaker operating at 200 rpm. After 96-120 hours, 4% of this culture is inoculated into a second series of flasks containing the same fermentation medium and grown for another 14 days under the same conditions. The production of antibiotic FIIRV 104/18 complex is monitored by HPLC as previously described, after extraction of the mycelium with the 2 mL ethanol per gram of mycelium. The extraction is performed at room temperature under stirring for two hours.

### EXAMPLE 3: RECOVERY OF ANTIBIOTIC FIIRV 104/18 COMPLEX

The fermentation broth described in Example 2 is centrifuged to obtain 8 L of supernatant and 2 L of mycelium. Antibiotic FIIRV 104/18 complex is found both in the supernatant (A) and in the mycelium (B) and these fractions are processed separately. Alternatively, the whole strain culture can be added with adsorbing resin and processed as in method (C).
(A) To the supernatant of the centrifuged broth 300 mL of Diaion HP-20 polystyrenic resin are added; the mixture is stirred 2 hours at room temperature and then the resin is recovered, washed with 1 L ethanol: water 1:3 (v/v) and then eluted twice with 1 L ethanol stirring overnight at room temperature. The pooled eluted fractions containing antibiotic FIIRV 104/18 complex are concentrated to small volume on a rotary evaporator and then diluted with 500 mL water. The resulting suspension is extracted with 2 × 500 mL of ethyl acetate. The ethyl acetate extract is washed with an equal volume of water followed by an equal volume of saturated solution of NaCl. The ethyl acetate extract is further dried by passing over a bed of anhydrous sodium sulfate. Antibiotic FIIRV 104/18 complex is then crystallized from the ethyl acetate solution by evaporation under vacuum to small volume and/or addition of excess petroleum ether to give precipitation, and then recovered by filtration.
(B) 250 mL of acetonitrile are first added to 1.2 kg of centrifuged biomass (mycelium) and allowed to contact for 30 min at room temperature, then 2.4 L of ethyl actetate are added and the supension is further stirred for 16h at room temperature. The liquid containing water, acetontrile and ethyl acetate is removed and concentrated to small volume on a rotary evaporator and then the resulting suspension is washed with 2 equal volumes of water to remove acetonitrile and then washed with an equal volume of a saturated NaCl solution. The washed ethyl acetate extract is then further dried with anhydrous sodium sulfate and the crude complex of factors is obtained after evaporation under vacuum.
(C) To 10 L of strain culture Diaion HP-20 polystyrenic resin (ratio 1:40 w/v) is added at the end of the fermentation period to adsorb the secreted secondary metabolites. The culture and resin are shaken at 215 r.p.m. for two additional hours. The resin and cell mass are collected by centrifugation or filtration through paper disks, and the residue is washed with deionized water to remove salts. The resin and cell mass are then resuspended and stirred with 2.5 L of ethanol for 2-4 hours at room temperature. The ethanol extract is centrifuged or filtered, the solid bulk is washed several times with 500 mL of ethanol and finally stirred with 500 mL of ethyl acetate for 30 minutes at room temperature, then filtered. Finally, the organic solvents are removed under vacuum to yield a crude extract. This is then processed as described in (B). The ethyl acetate extract is washed with an equal volume of water followed by an equal volume of saturated solution of NaCl. The ethyl acetate extract is further dried by passing over a bed of anhydrous sodium sulfate, then evaporated under vacuum.

### EXAMPLE 4: RECOVERY OF ANTIBIOTIC FIIRV 104/18 INDIVIDUAL FACTORS: F793, F795, F797, F813 AND OF COMPARATIVE FACTOR F683

The individual factors may be separated from the antibiotic FIIRV 104/18 complex, prepared according to the procedure described in Example 3, by using the preparative HPLC method described in Example 1 (phase A: 20mM ammonium formate pH 4.5, phase B: CH₃CN). The separation is performed by using a column (250 × 21.2 mm) Phenomenex Luna-C18(2) (Phenomenex, Torrance CA). The antibiotic FIIRV 104/18 complex is dissolved in DMSO: H₂O:CH₃CN 1:1:1 (v/v) . In these conditions, factors F683 and F813 co-elute with 40% of phase B and show a rt of 13.0 min; pure factor F797 elutes with 42% phase B at rt 17.5 min, factor F795 elutes with 48% phase B at 27.5 min and factor F793 elutes with 50% phase B at 30.5 min. The eluted fractions containing pure individual antibiotic FIIRV 104/18 factors F797, F795 and F793 are collected and concentrated under vacuum to aqueous phase. The water solutions are extracted with ethyl acetate and concentrated to minimal volume; petroleum ether is added and pure factors are precipitated and filtered. From 1.2 kg of mycelium, 175.5 mg of factor F795, 128.5 mg of factor F797 and 8.7 mg of factor F793 are recovered.

Factors F683 and F813 are separated and purified from the above reported mixed fractions by preparative HPLC using a Phenomenex Luna Pheny Hexyl 5µ (250 × 10 mm) column. A sample of antibiotic FIIRV 104/18 F813 and of factor F683 mixture is dissolved in DMSO:H₂O:CH₃CN 1:1:1 (v/v) and a 25 minutes linear gradient elution from 25% to 40% of phase B at 8 mL flow rate is performed. Phase B is CH₃CN. Phase A is ammonium formate 20 mM pH4.5. Pure factor F683 elutes at 16.5 min rt with 36% phase B, and pure factor F813 elutes at 17.5 min rt with 37% phase B. The eluted fractions containing pure antibiotic FIIRV 104/18 factors F683 and F813 are collected and concentrated to aqueous phase under vacuum. Pure factors are obtained by extracting the aqueous phase with ethyl acetate, concentrating to minimal volume said extract, adding petroleum ether thereto and recovering the precipitated product by filtration. From 1.2 kg of mycelium, 11.1 mg of pure F683 and 2.7 mg of F813 are recovered.

## Claims

1. An antibiotic FIIRV 104/18 complex comprising factors F793, F795, F797, and F813, of formula (I): 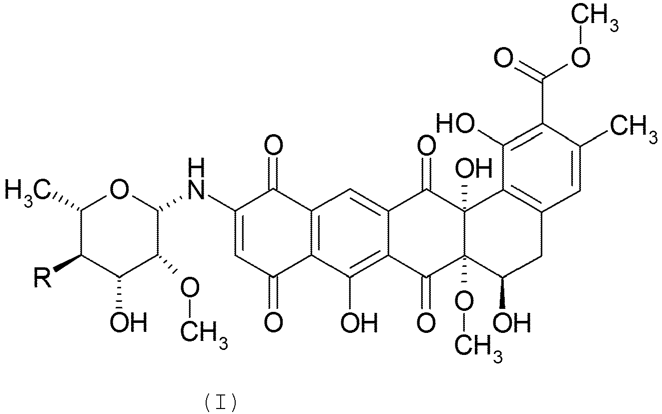 wherein R represents 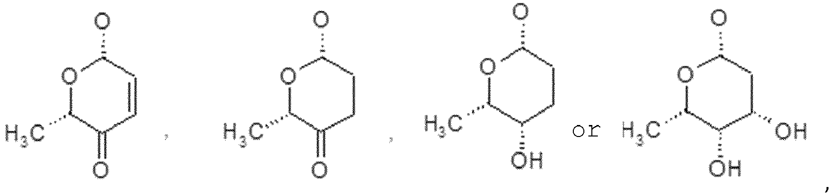 or the isolated individual factors thereof, or a mixture of two or more of said isolated individual factors.

2. An isolated factor of the antibiotic FIIRV 104/18 complex according to claim 1, selected from the group consisting of:
factor F793 of formula 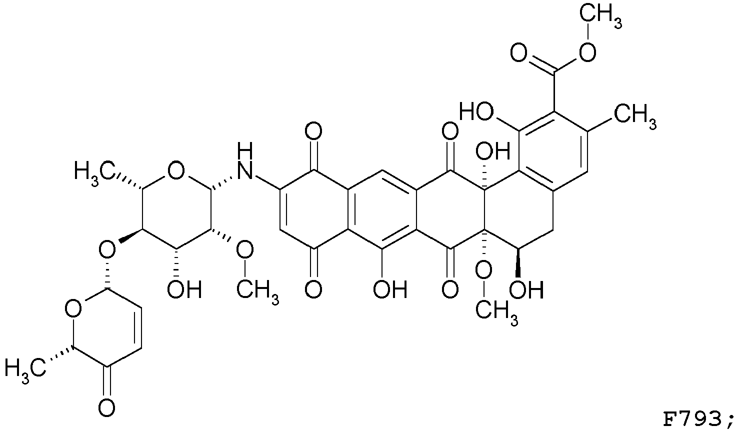
factor F795 of formula 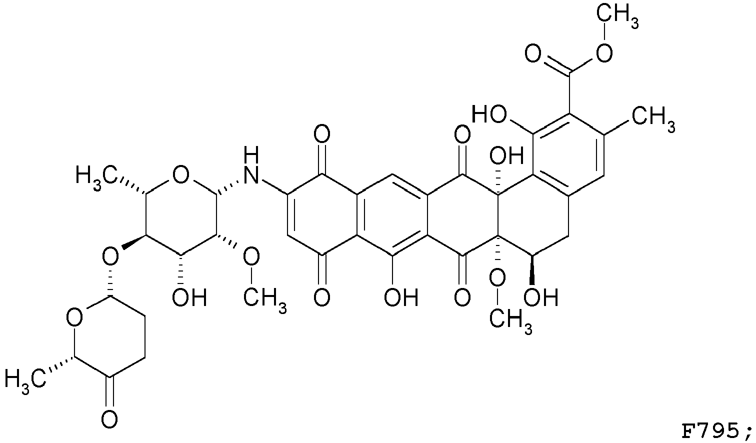
factor F797 of formula 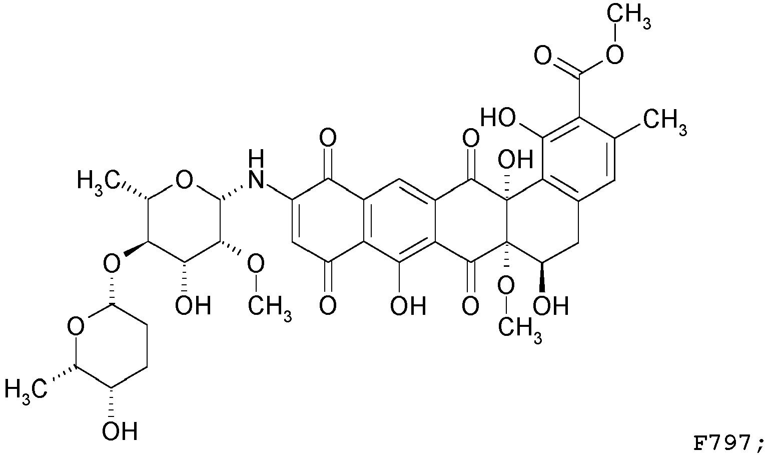
and factor F813 of formula

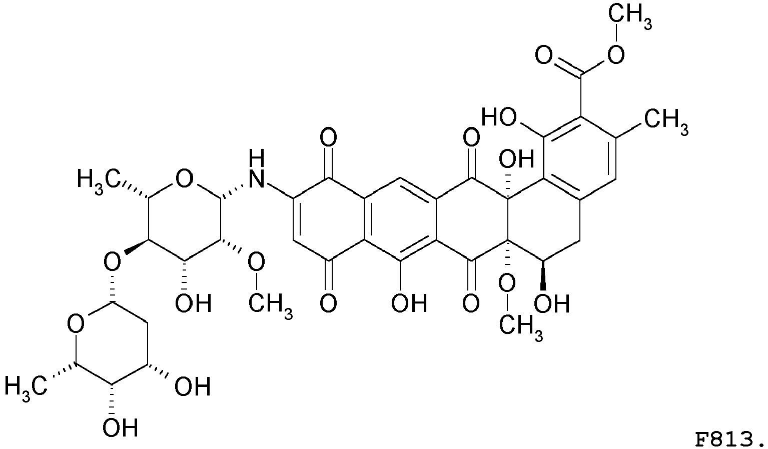

3. A process for the preparation of the antibiotic FIIRV 104/18 complex of formula (I) according to claim 1, comprising:
(a) cultivating *Dactylosporangium* FIIRV sp. 104/18 DSM 32068, under aerobic conditions, in an aqueous nutrient medium containing usable sources of carbon, nitrogen and inorganic salts;
(b) isolating the resulting antibiotic of formula (I) from the whole fermentation broth, or from the separated mycelium or from the filtered or centrifuged fermentation broth;
(c) purifying the isolated antibiotic of formula (I).

4. The process according to claim 3, wherein the strain *Dactylosporangium FIIRV sp. 104*/*18 DSM 32068* is pre-cultured.

5. The process according to any one of claim 3 and 4, wherein the isolation of the antibiotic FIIRV 104/18 complex of claim 1 is carried out by centrifuging or filtering the fermentation broth and recovering the antibiotic from the supernatant or filtered broth according to at least one technique selected from the group consisting of: extraction with a water- immiscible solvent, precipitation by adding a non-solvent or by changing the pH of the solution, absorption chromatography, partition chromatography, reverse phase partition chromatography, ion exchange chromatography and molecular exclusion chromatography, or a combination of two or more of said techniques.

6. The process according to any one of claim 3 and 4, wherein the isolation of the antibiotic FIIRV 104/18 complex of claim 1 is carried out by separating the mycelium from the supernatant of the fermentation broth and extracting the mycelium with a water-miscible solvent whereby, after the removal of the spent mycelium, a water-miscible solution containing the crude antibiotic is obtained, which is processed to recover the antibiotic FIIRV 104/18 complex by means of at least one technique selected from the group consisting of: extraction with a water-miscible solvent, precipitation by adding a non-solvent or by changing the pH of the solution, absorption chromatography, partition chromatography, reverse phase partition chromatography, ion exchange chromatography and molecular exclusion chromatography, or a combination of two or more of said techniques.

7. The process according to any one of claim 3 and 4, wherein the isolation of the antibiotic FIIRV 104/18 complex of claim 1 is carried out by separating the mycelium from the supernatant of the fermentation broth and extracting the mycelium with a water-immiscible solvent whereby, after the removal of the spent mycelium, a solution containing the crude antibiotic is obtained, which is subjected to further purification steps.

8. The process according to any one of claim 3 and 4, wherein the isolation of the antibiotic FIIRV 104/18 complex of claim 1 is carried out on the whole microbial culture by adding an absorbing resin, maintaining the obtained mixture under stirring until the antibiotic product is almost entirely bound to the resin, separating the resin and mycelium by filtration or centrifugation and submitting said separated material to extraction with a water-miscible solvent, thus obtaining a solution of the antibiotic FIIRV 104/18 complex from which the product is recovered by at least one of the techniques mentioned in claim 6.

9. The process according to any one of claims 3 to 8, wherein the individual factors F793, F795, F797, and F813 are separated from the antibiotic FIIRV 104/18 complex by using a preparative HLPC method.

10. A pharmaceutical composition comprising the antibiotic FIIRV 104/18 complex comprising factors F793, F795, F797, and F813 or an isolated individual factor thereof, or a mixture of two or more of said isolated individual factors according to claim 1.

11. The pharmaceutical composition according to claim 10, further comprising a pharmaceutically acceptable carrier; preferably wherein the composition is in orally, topically, or parenterally administrable form; more preferably wherein the composition is in the form of a capsule, a tablet, a lozenge, a liquid solution, a liquid suspension, an aqueous solution, an aqueous suspension, an oily solution, an oily suspension, a hydrophobic base ointment, a hydrophobic base cream, a hydrophobic base lotion, a hydrophobic base paint, a hydrophobic base powder, a hydrophilic base ointment, a hydrophilic base cream, a hydrophilic base lotion, a hydrophilic base paint, and a hydrophilic base powder.

12. Antibiotic FIIRV 104/18 complex comprising factors F793, F795, F797, and F813 or an isolated individual factor thereof or a mixture of two or more of said isolated individual factors according to any one of claim 1 to 2, for use as medicament for the treatment of a bacterial infection.

13. The antibiotic FIIRV 104/18 complex comprising factors F793, F795, F797, and F813 or an isolated individual factor thereof or a mixture of two or more of said isolated individual factors for use as a medicament according to claim 12, wherein said bacterial infection is caused by bacteria of at least one of the genera selected from the group consisting of: Enterococci, Streptococci, Staphylococci and Moraxella; preferably wherein said bacterial infection is caused by bacteria selected from the multidrug-resistant strains of Staphylococcus spp., Streptococcus spp., and Enterococcus spp.; more preferably wherein said bacterial infection is caused by bacteria selected from the multidrug-resistant strains of Staphylococcus spp., Streptococcus spp, and Enterococcus spp. resistant to methicillin and/or vancomycin.

14. The pharmaceutical composition of claim 10 for use according to claim 12, wherein the dosage range is comprised between 1 and 40 mg of active ingredient per Kg of body weight.

15. A biologically pure culture of the strain *Dactylosporangium* FIIRV sp. 104/18 DSM 32068.

## Patentansprüche

1. Antibiotischer FIIRV 104/18-Komplex, umfassend Faktoren F793, F795, F797 und F813, der Formel (I): 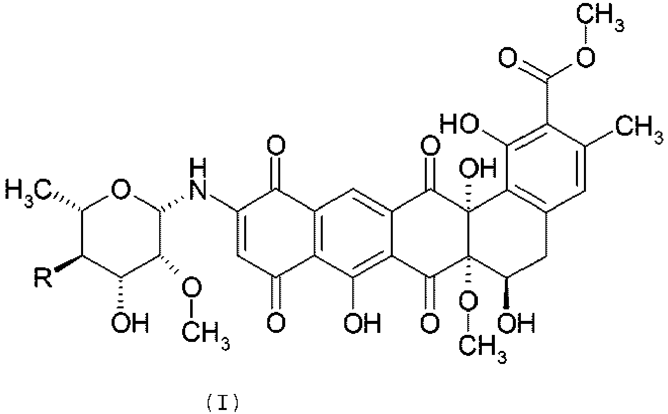 wobei R Folgendes darstellt 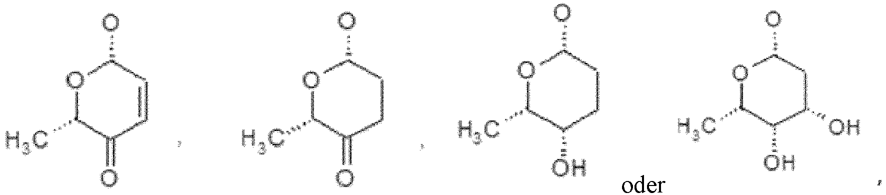 oder die isolierten individuelle Faktoren davon oder eine Mischung aus zwei oder mehr der isolierten individuellen Faktoren.

2. Isolierter Faktor des antibiotischen FIIRV 104/18-Komplexes nach Anspruch 1, ausgewählt aus der Gruppe, die aus Folgendem besteht:
Faktor F793 der folgenden Formel 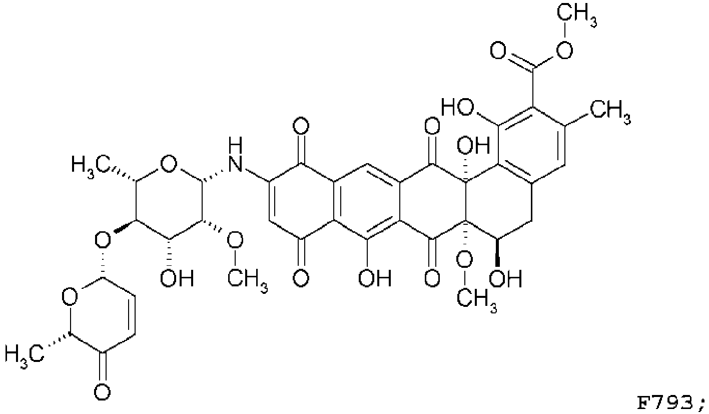
Faktor F795 der folgenden Formel 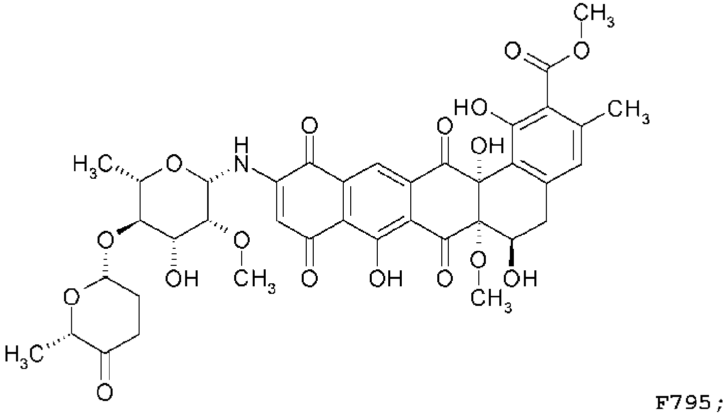
Faktor F797 der folgenden Formel 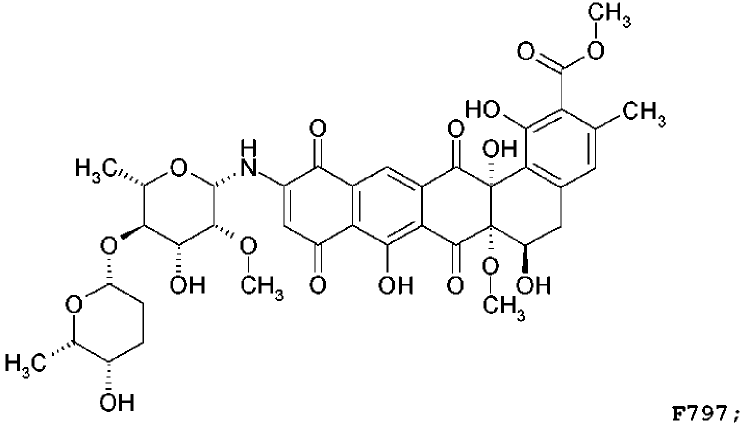
und Faktor F813 der folgenden Formel

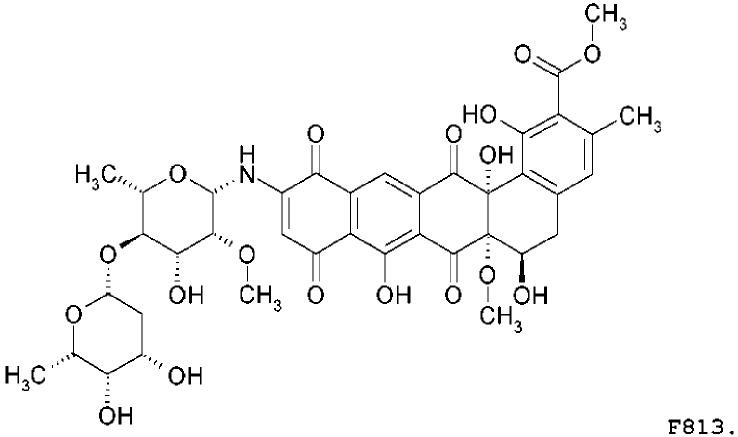

3. Verfahren zur Herstellung des antibiotischen FIIRV 104/18-Komplexes der Formel (I) nach Anspruch 1, umfassend:
(a) Kultivieren von *Dactylosporangium* FIIRV sp. 104/18 DSM 32068 unter aeroben Bedingungen in einem wässrigen Nährmedium, das verwertbare Quellen für Kohlenstoff, Stickstoff und anorganische Salze enthält;
(b) Isolieren des resultierenden Antibiotikums der Formel (I) aus der gesamten Fermentationsbrühe oder aus dem abgetrennten Myzel oder aus der gefilterten oder zentrifugierten Fermentationsbrühe;
(c) Reinigen des isolierten Antibiotikums der Formel (I).

4. Verfahren nach Anspruch 3, wobei der Stamm *Dactylosporangium FIIRVsp. 104*/*18 DSM 32068* vorkultiviert ist.

5. Verfahren nach einem der Ansprüche 3 und 4, wobei die Isolierung des antibiotischen FIIRV 104/18-Komplexes von Anspruch 1 durch Zentrifugieren oder Filtern der Fermentationsbrühe und Gewinnen des Antibiotikums aus dem Überstand oder der gefilterten Brühe gemäß mindestens einer Technik durchgeführt wird, die aus der Gruppe ausgewählt ist, die aus Folgendem besteht: Extraktion mit einem mit Wasser nicht mischbaren Lösungsmittel, Ausfällung durch Zugeben eines Nichtlösungsmittels oder durch Ändern des pH-Werts der Lösung, Absorptionschromatographie, Verteilungschromatographie, Umkehrphasenverteilungschromatographie, Ionenaustauschchromatographie und Molekularausschlusschromatographie oder eine Kombination aus zwei oder mehr der Techniken.

6. Verfahren nach einem von Anspruch 3 und 4, wobei die Isolierung des antibiotischen FIIRV 104/18-Komplexes von Anspruch 1 durch Abtrennen des Myzels von dem Überstand der Fermentationsbrühe und Extrahieren des Myzels mit einem mit Wasser mischbaren Lösungsmittel durchgeführt wird, wodurch nach der Entfernung des verbrauchten Myzels eine mit Wasser mischbare Lösung, die das rohe Antibiotikum enthält, erhalten wird, die verarbeitet wird, um den antibiotischen FIIRV 104/18-Komplex mittels mindestens einer Technik zu gewinnen, die aus der Gruppe ausgewählt ist, die aus Folgendem besteht: Extraktion mit einem mit Wasser mischbaren Lösungsmittel, Ausfällung durch Zugeben eines Nichtlösungsmittels oder durch Ändern des pH-Werts der Lösung, Absorptionschromatographie, Verteilungschromatographie, Umkehrphasenverteilungschromatographie, Ionenaustauschchromatographie und Molekularausschlusschromatographie oder eine Kombination aus zwei oder mehr der Techniken.

7. Verfahren nach einem von Anspruch 3 und 4, wobei die Isolierung des antibiotischen FIIRV 104/18-Komplexes von Anspruch 1 durch Abtrennen des Myzels von dem Überstand der Fermentationsbrühe und Extrahieren des Myzels mit einem mit Wasser nicht mischbaren Lösungsmittel durchgeführt wird, wodurch nach der Entfernung des verbrauchten Myzels eine Lösung, die das rohe Antibiotikum enthält, erhalten wird, die weiteren Reinigungsschritten unterzogen wird.

8. Verfahren nach einem von Anspruch 3 und 4, wobei die Isolierung des antibiotischen FIIRV 104/18-Komplexes von Anspruch 1 an der gesamten mikrobiellen Kultur durch Zugeben eines absorbierenden Harzes, Weiterrühren der erhaltenen Mischung, bis das antibiotische Produkt fast vollständig an das Harz gebunden ist, Abtrennen des Harzes und des Myzels durch Filtration oder Zentrifugation und Aussetzen des abgetrennten Materials einer Extraktion mit einem mit Wasser mischbaren Lösungsmittel durchgeführt wird, wodurch eine Lösung des antibiotischen FIIRV 104/18-Komplexes erhalten wird, aus der das Produkt durch mindestens eine der in Anspruch 6 genannten Techniken gewonnen wird.

9. Verfahren nach einem der Ansprüche 3 bis 8, wobei die individuellen Faktoren F793, F795, F797 und F813 unter Verwendung einer präparativen HLPC-Methode von dem antibiotischen FIIRV 104/18-Komplex abgetrennt werden.

10. Pharmazeutische Zusammensetzung, umfassend den antibiotischen FIIRV 104/18-Komplex, der die Faktoren F793, F795, F797 und F813 umfasst, oder einen isolierten individuellen Faktor davon oder eine Mischung aus zwei oder mehreren der isolierten individuellen Faktoren nach Anspruch 1.

11. Pharmazeutische Zusammensetzung nach Anspruch 10, ferner einen pharmazeutisch unbedenklichen Träger umfassend; wobei die Zusammensetzung bevorzugt in einer oral, topisch oder parenteral verabreichbaren Form vorliegt; wobei die Zusammensetzung besonders bevorzugt in der Form einer Kapsel, einer Tablette, einer Lutschtablette, einer flüssigen Lösung, einer flüssigen Suspension, einer wässrigen Lösung, einer wässrigen Suspension, einer öligen Lösung, einer öligen Suspension, einer hydrophoben Basissalbe, einer hydrophoben Basiscreme, einer hydrophoben Basislotion, einer hydrophoben Basisfarbe, eines hydrophoben Basispuders, einer hydrophilen Basissalbe, einer hydrophilen Basiscreme, einer hydrophilen Basislotion, einer hydrophilen Basisfarbe und eines hydrophilen Basispuders vorliegt.

12. Antibiotischer FIIRV 104/18-Komplex, umfassend die Faktoren F793, F795, F797 und F813, oder ein isolierter individueller Faktor davon oder eine Mischung aus zwei oder mehreren der isolierten individuellen Faktoren nach einem der Ansprüche 1 bis 2, zur Verwendung als Medikament zur Behandlung einer bakteriellen Infektion.

13. Antibiotischer FIIRV 104/18-Komplex, umfassend die Faktoren F793, F795, F797 und F813, oder ein isolierter individueller Faktor davon oder eine Mischung aus zwei oder mehreren der isolierten individuellen Faktoren zur Verwendung als ein Medikament nach Anspruch 12, wobei die bakterielle Infektion durch Bakterien von mindestens einer der Gattungen verursacht wird, die aus der Gruppe ausgewählt sind, die aus Folgenden besteht: Enterokokken, Streptokokken, Staphylokokken und Moraxella; wobei die bakterielle Infektion bevorzugt durch Bakterien verursacht wird, die aus den multiresistenten Stämmen von Staphylococcus spp., Streptococcus spp. und Enterococcus spp. ausgewählt sind; wobei die bakterielle Infektion besonders bevorzugt durch Bakterien verursacht wird, die aus den multiresistenten Stämmen von Staphylococcus spp., Streptococcus spp. und Enterococcus spp. ausgewählt sind, die gegen Methicillin und/oder Vancomycin resistent sind.

14. Pharmazeutische Zusammensetzung nach Anspruch 10 zur Verwendung nach Anspruch 12, wobei der Dosierungsbereich zwischen 1 und 40 mg Wirkstoff pro kg Körpergewicht umfasst ist.

15. Biologisch reine Kultur des Stammes *Dactylosporangium* FIIRV sp. 104/18 DSM 32068.

## Revendications

1. Complexe antibiotique FIIRV 104/18 comprenant les facteurs F793, F795, F797 et F813, de formule (I) : 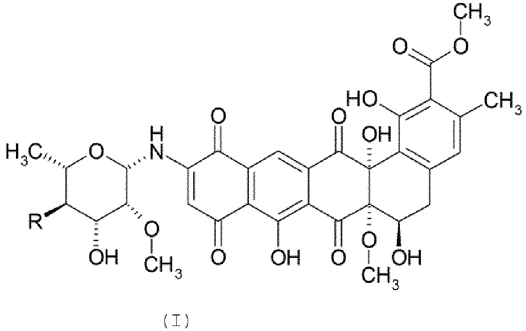
dans laquelle R représente 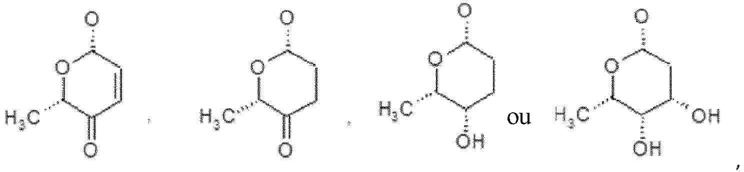
ou les facteurs individuels isolés de celui-ci, ou un mélange de deux desdits facteurs individuels isolés ou plus.

2. Facteur individuel isolé du complexe antibiotique FIIRV 104/18 selon la revendication 1, choisi dans le groupe constitué par :
le facteur F793 de formule 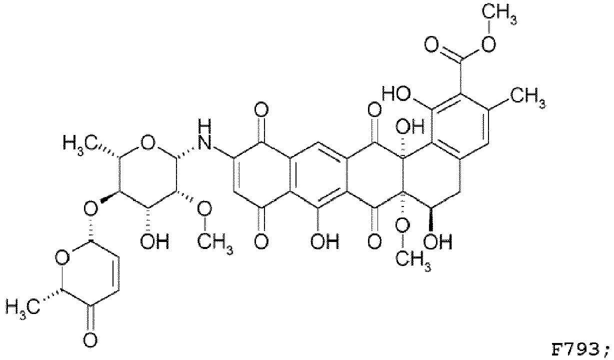
le facteur F795 de formule 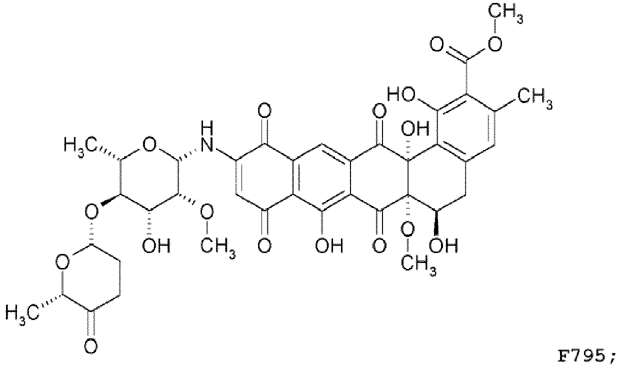
le facteur F797 de formule 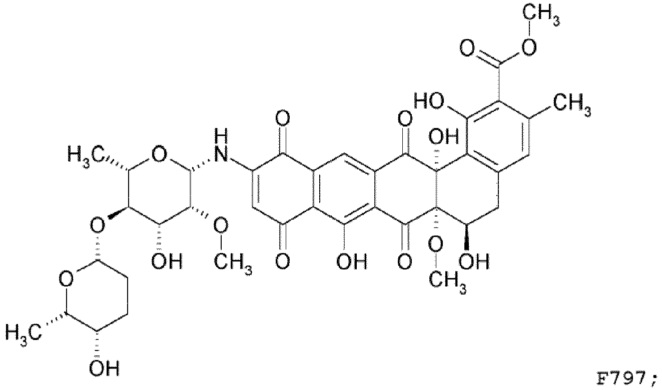
et le facteur F813 de formule

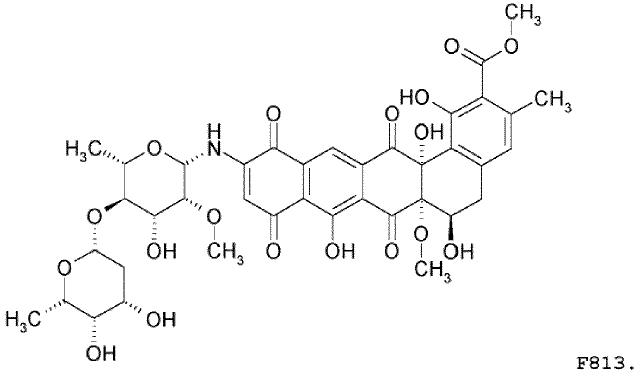

3. Procédé de préparation du complexe antibiotique FIIRV 104/18 de formule (I) selon la revendication 1, comprenant :
(a) la culture du *Dactylosporangium* FIIRV sp. 104/18 DSM 32068, dans des conditions aérobies, dans un milieu nutritif aqueux contenant des sources utilisables de carbone, d'azote et de sels inorganiques ;
(b) l'isolement de l'antibiotique résultant de formule (I) à partir du bouillon de fermentation complet, ou à partir du mycélium séparé ou à partir du bouillon de fermentation filtré ou centrifugé ;
(c) la purification de l'antibiotique de formule (I) isolé.

4. Procédé selon la revendication 3, la souche *Dactylosporangium FIIRV sp. 104*/*18 DSM 32068* étant précultivée.

5. Procédé selon l'une quelconque des revendications 3 et 4, ledit isolement du complexe antibiotique FIIRV 104/18 selon la revendication 1 étant effectué par centrifugation ou filtration du bouillon de fermentation et récupération de l'antibiotique à partir du surnageant ou du bouillon filtré conformément à au moins une technique choisie dans le groupe constitué par : l'extraction avec un solvant non miscible dans l'eau, la précipitation par ajout d'un non-solvant ou par changement du pH de la solution, la chromatographie par absorption, la chromatographie de partage, la chromatographie de partage en phase inversée, la chromatographie échangeuse d'ions et la chromatographie d'exclusion moléculaire, ou une combinaison de deux desdites techniques ou plus.

6. Procédé selon l'une quelconque des revendications 3 et 4, ledit isolement du complexe antibiotique FIIRV 104/18 selon la revendication 1 étant effectué par séparation du mycélium à partir du surnageant du bouillon de fermentation et extraction du mycélium avec un solvant miscible dans l'eau grâce à quoi, après retrait du mycélium usagé, une solution miscible dans l'eau contenant l'antibiotique brut est obtenue, qui est traitée pour récupérer le complexe antibiotique FIIRV 104/18 au moyen d'au moins une technique choisie dans le groupe constitué par : l'extraction avec un solvant miscible dans l'eau, la précipitation par ajout d'un non-solvant ou par changement du pH de la solution, la chromatographie par absorption, la chromatographie de partage, la chromatographie de partage en phase inversée, la chromatographie échangeuse d'ions et la chromatographie d'exclusion moléculaire, ou une combinaison de deux desdites techniques ou plus.

7. Procédé selon l'une quelconque des revendications 3 et 4, ledit isolement du complexe antibiotique FIIRV 104/18 selon la revendication 1 étant effectué par séparation du mycélium à partir du surnageant du bouillon de fermentation et extraction du mycélium avec un solvant non miscible dans l'eau grâce à quoi, après retrait du mycélium usagé, une solution contenant l'antibiotique brut est obtenue, qui est soumise à des étapes de purification supplémentaires.

8. Procédé selon l'une quelconque des revendications 3 et 4, ledit isolement du complexe antibiotique FIIRV 104/18 selon la revendication 1 étant effectué sur la culture microbienne complète par ajout d'une résine absorbante, maintien du mélange obtenu sous agitation jusqu'à ce que le produit antibiotique soit presque entièrement lié à la résine, séparation de la résine et du mycélium par filtration ou centrifugation et soumission de ladite matière séparée à une extraction avec un solvant miscible dans l'eau, obtenant ainsi une solution du complexe antibiotique FIIRV 104/18 à partir de laquelle le produit est récupéré par au moins l'une des techniques mentionnées dans la revendication 6.

9. Procédé selon l'une quelconque des revendications 3 à 8, lesdits facteurs individuels F793, F795, F797 et F813 étant séparés du complexe antibiotique FIIRV 104/18 à l'aide d'une méthode HPLC préparative.

10. Composition pharmaceutique comprenant le complexe antibiotique FIIRV 104/18 comprenant les facteurs F793, F795, F797 et F813 ou un facteur individuel isolé de ceux-ci, ou un mélange de deux desdits facteurs individuels isolés ou plus selon la revendication 1.

11. Composition pharmaceutique selon la revendication 10, comprenant en outre un vecteur pharmaceutiquement acceptable ; de préférence ladite composition se présentant sous une forme administrable par voie orale, topique ou parentérale ; plus préférablement ladite composition se présentant sous la forme d'une capsule, d'un comprimé, d'une pastille, d'une solution liquide, d'une suspension liquide, d'une solution aqueuse, d'une suspension aqueuse, d'une solution huileuse, d'une suspension huileuse, d'un onguent à base hydrophobe, d'une crème à base hydrophobe, d'une lotion à base hydrophobe, d'une peinture à base hydrophobe, d'une poudre à base hydrophobe, d'un onguent à base hydrophile, d'une crème à base hydrophile, d'une lotion à base hydrophile, d'une peinture à base hydrophile, et d'une poudre à base hydrophile.

12. Complexe antibiotique FIIRV 104/18 comprenant les facteurs F793, F795, F797 et F813 ou un facteur individuel isolé de ceux-ci, ou un mélange de deux desdits facteurs individuels isolés ou plus selon l'une quelconque des revendications 1 à 2, destiné à être utilisé en tant que médicament pour le traitement d'une infection bactérienne.

13. Complexe antibiotique FIIRV 104/18 comprenant les facteurs F793, F795, F797 et F813 ou un facteur individuel isolé de ceux-ci, ou un mélange de deux desdits facteurs individuels isolés ou plus, destiné à être utilisé selon la revendication 12, ladite infection bactérienne étant causée par des bactéries d'au moins l'un des genres choisi dans le groupe constitué par : les entérocoques, les streptocoques, les staphylocoques et les moraxelles ; de préférence ladite infection bactérienne étant causée par des bactéries choisies parmi les souches résistantes à de multiples médicaments de Staphylococcus spp., Streptococcus spp., et d'Enterococcus spp. ; plus préférablement ladite infection bactérienne étant causée par des bactéries choisies parmi les souches résistantes à de multiples médicaments de Staphylococcus spp., de Streptococcus spp., et d'Enterococcus spp. résistantes à la méthicilline et/ou à la vancomycine.

14. Composition pharmaceutique selon la revendication 10 destinée à être utilisée selon la revendication 12, la plage posologique étant comprise entre 1 et 40 mg de principe actif par kg de poids corporel.

15. Culture biologiquement pure de la souche *Dactylosporangium* FIIRV sp. 104/18 DSM 32068.
